(19) Europäisches Patentamt
European Patent Office
Office européen des brevets

(11) **EP 4 077 695 B1**

(12) **EUROPEAN PATENT SPECIFICATION**

(45) Date of publication and mention
of the grant of the patent:
**24.07.2024 Bulletin 2024/30**

(21) Application number: **20823810.5**

(22) Date of filing: **14.12.2020**

(51) International Patent Classification (IPC):
**C12P 13/04** *(2006.01)*   **C12N 9/00** *(2006.01)*
**C12N 9/10** *(2006.01)*

(52) Cooperative Patent Classification (CPC):
**C12N 9/1003; C12P 13/04; C12Y 201/01002;**
**C12Y 201/03003; C12Y 201/04001;**
**C12Y 403/02001; C12Y 603/04005;**
**C12Y 603/04016; C12Y 603/05005**

(86) International application number:
**PCT/EP2020/085882**

(87) International publication number:
**WO 2021/122400 (24.06.2021 Gazette 2021/25)**

(54) **METHOD FOR THE FERMENTATIVE PRODUCTION OF GUANIDINOACETIC ACID**

VERFAHREN ZUR FERMENTATIVEN HERSTELLUNG VON GUANIDINOESSIGSÄURE

PROCÉDÉ DE PRODUCTION D'ACIDE GUANIDINOACETIQUE PAR FERMENTATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.12.2019 EP 19218204**

(43) Date of publication of application:
**26.10.2022 Bulletin 2022/43**

(73) Proprietor: **Evonik Operations GmbH**
**45128 Essen (DE)**

(72) Inventors:
• **SCHNEIDER, Frank**
**33790 Halle (DE)**
• **JANKOWITSCH, Frank**
**48336 Sassenberg (DE)**

(74) Representative: **Evonik Patent Association**
**c/o Evonik Industries AG**
**IP Management**
**Bau 1042A/PB 15**
**Paul-Baumann-Straße 1**
**45772 Marl (DE)**

(56) References cited:
**WO-A1-2006/057450    WO-A1-2018/079687**
**US-A1- 2006 200 870**

• **DATABASE UniProt [online] 15 February 2017
(2017-02-15), "Glycine amidinotransferase",
XP055706853, retrieved from EBI accession no.
UNIPROT:A0A1D8TKD3 Database accession no.
A0A1D8TKD3**
• **DATABASE UniProt [online] 16 January 2019
(2019-01-16), "Glycine amidinotransferase",
XP055787003, retrieved from EBI accession no.
UNIPROT:A0A3F3KLT7 Database accession no.
A0A3F3KLT7**
• **DATABASE NCBI [online] NCBI; 22 July 2014
(2014-07-22), "PREDICTED: glycine
amidinotransferase, mitochondrial [Galeopterus
vari - Protein - NCBI", XP055787022, retrieved
from
https://www.ncbi.nlm.nih.gov/protein/XP_00858
3331 Database accession no. NCBI:
XP_008583331**
• **DATABASE UniProt [online] 1 October 1996
(1996-10-01), "L-arginine:glycine
amidinotransferase", XP002802407, retrieved
from EBI accession no. UNIPROT:P50440
Database accession no. P50440**

EP 4 077 695 B1

- **MARC F ET AL: "Characterization and kinetic mechanism of mono- and bifunctional ornithine acetyltransferases from thermophilic microorganisms", EUROPEAN JOURNAL OF BIOCHEMISTRY, WILEY-BLACKWELL PUBLISHING LTD, GB, vol. 267, no. 16, 1 August 2000 (2000-08-01), pages 5217 - 5226, XP002169947, ISSN: 0014-2956, DOI: 10.1046/J.1432-1327.2000.01593.X**

Remarks:

The complete document including Reference Table(s) and the Sequence Listing(s) can be downloaded from the EPO website

**Description**

The present invention relates to a microorganism transformed to be capable of producing guanidinoacetic acid (GAA) and to a method for the fermentative production of GAA using such a microorganism. The present invention also relates to a method for the fermentative production of creatine.

GAA is an organic compound used as animal feed additive (WO2005120246 A1/ US2011257075 A1). GAA is a natural precursor of creatine (e.g. Humm et al., Biochem. J. (1997) 322, 771-776). Therefore, the supplementation of GAA allows for an optimal supply of creatine in the organism.

The present invention pertains to a method to produce GAA by a fermentative process using industrial feed stocks (e.g. ammonia, ammonium salts and glucose or sugar containing substrates) as starting material. In biological systems GAA and ornithine are formed from arginine and glycine as starting materials by the catalytic action of an L-arginine:glycine-amidinotransferase (AGAT; EC 2.1.4.1), which is the first step in creatine biosynthesis (US 20060200870 A1):

$$\text{L-arginine} + \text{glycine} \xrightarrow{\underline{\textbf{AGAT}}} \text{L-ornithine} + \text{GAA}$$

Guthmiller et al. (J Biol Chem. 1994 Jul 1;269(26):17556-60) have characterized a rat kidney AGAT by cloning and heterologously expressing the enzyme in *E. coli.* Muenchhoff et al. (FEBS Journal 277 (2010) 3844-3860) report the first characterization of an AGAT from a prokaryote also by cloning and heterologously expressing the enzyme in *E. coli.* Sosio et al. (Cell Chemical Biology 25, 540-549, May 17, 2018) elucidated the biosynthetic pathway for pseudouridimycin in *Streptomyces* sp.. They describe as an intermediate reaction the formation of GAA and L-ornithine by the reaction of L-arginine with glycine catalyzed by PumN, an L-arginine:glycine-amidinotransferase (AGAT). Humm et al. expressed a recombinant gene encoding human AGAT in *Escherichia coli* and identified cysteine-407 as an active-site residue of AGAT (Biochem. J. (1997) 322, 771-776).

Mijts et al. (WO 2018079687 A1) disclose in the context of the production of objective substances in microorganisms, e.g. vanillin and vanillic acid, that creatine can be produced from L-arginine and glycine. The authors further propose that this may be achieved by using L-arginine biosynthesis enzymes, glycine biosynthesis enzymes, and enzymes that catalyse the conversion of L-arginine and glycine into creatine. L-arginine and glycine can be combined to generate guanidinoacetate (GAA) and ornithine by the action of AGAT (EC 2.1.4.1) and that GAA can be methylated to generate creatine by the action of guanidinoacetate N-methyltransferase (GAMT, EC 2.1.1.2), using S-adenosyl methionine (SAM) as the methyl donor. The authors mention in the context of the production of polyamines that examples for L-arginine biosynthesis enzymes may also include the well-known L-ornithine biosynthesis enzymes, as well as the enzymes that are well-known from the so called L-ornithine cycle, i.e. carbamoyl phosphate synthase (*carAB*), ornithine carbamoyl transferase (*argF, argl*), argininosuccinate synthetase (*argG*), argininosuccinate lyase (*argH*) (cf. Marc et al., Eur. J. Biochem. 267, 5217-5226, 200).

However, several more specific approaches for increasing the production of one of the starting materials in GAA synthesis, i.e. L-arginine, in microorganisms, particularly bacteria, are also known from literature. An overview for the metabolic engineering of *Corynebacterium glutamicum* (*C. glutamicum)* for L-arginine production is provided by Park et al. (NATURE COMMUNICATIONS | DOI: 10.1038/ncomms5618). They propose random mutagenesis and screening for L-arginine producers of already L-arginine producing C. *glutamicum* strains, e.g. ofATCC 21831 (Nakayama and Yoshida 1974, US3849250 A) and stepwise rational metabolic engineering based on system-wide analysis of metabolism results in a gradual increase in L-arginine production throughout the strain engineering steps. Yim et al. (J Ind Microbiol Biotechnol (2011) 38:1911-1920) could show that inactivation of the *argR,* gene coding for the central repressor protein ArgR controlling the L-arginine biosynthetic pathway, by disrupting the chromosomal *argR* gene in *C. glutamicum* leads to an improved arginine-producing strain. Ginesy et al. (Microbial Cell Factories (2015) 14:29) report the successful engineering of *E. coli* for enhanced arginine production. Among other, they proposed the deletion of the *argR* repressor gene.

Kurahashi et al. (EP1057893 A1) report on methods for increasing the L-arginine producing ability of a microorganism by enhancing L-arginine biosynthesis enzymes utilizing recombinant DNA techniques, e.g. by utilizing a microorganism belonging to the genus *Corynebacterium* or *Brevibacterium* which is made to harbor a recombinant DNA comprising a vector DNA and a DNA fragment containing genes for acetylornithine deacetylase, N-acetylglutamic acid-γ-semialdehyde dehydrogenase, N-acetyl glutamokinase and argininosuccinase derived from a microorganism belonging to the genus *Escherichia.* For an improved L-arginine production the authors further propose a microorganism which is enhanced in an activity of intracellular glutamate dehydrogenase (GDH) and which has an L-arginine producing ability.

A method of using a genetic recombinant strain, wherein a gene which inhibits the expression of arginine-biosynthesizing operon, *argR,* was inactivated has been reported by Suga et al. (US7160705 B2). In particular, the deletion in *argR,* which controls the arginine operon, has been considered as an important factor in arginine production.

In a microorganism of *Corynebacterium,* the *argCJBDFR* gene, which is involved in arginine biosynthesis, is constituted in the form of an operon and is subjected to feedback-inhibition by intracellular arginine (Sakanyan et al., Microbiology, 142:9-108, 1996), thus imposing a limitation on its high yield L-arginine production.

However, Bae et al. (EP3153573 A1) in an attempt to increase the production yield of L-arginine in *C. glutamicum,* discovered that L-arginine can be produced in higher yield compared to the parental L-arginine-producing strain, by enhancing the activities of the arginine operon and ornithine carbamoyltransferase (ArgF, ArgF2), without any deletion in arginine repressor (*argR*).

The arginine operon is an operon consisting of genes encoding enzymes involved in the mechanism of L-arginine biosynthesis, and in particular, arginine operon consists of genes encoding enzymes constituting the cyclic steps of L-arginine biosynthesis. Specifically, the arginine operon consists of N-acetylglutamyl phosphate reductase (ArgC), glutamate N-acetyltransferase (ArgJ), N-acetylglutamate kinase (ArgB), acetylornithine aminotransferase (ArgD), ornithine carbamoyltransferase (ArgF), and the arginine repressor (ArgR). These enzymes are involved in the continuous enzyme reactions of L-arginine biosynthesis and are controlled by the arginine repressor encoded by *argR* (WO 2006/057450 A1).

Fan Wenchao discloses a method for the production of creatine by fermentation of non-pathogenic microorganisms, such as *Corynebacterium glutamicum* (CN106065411 A). The microorganism has the following biotransformation functions: glucose conversion to L-glutamic acid; conversion of L-glutamic acid to N-acetyl-L-glutamic acid; conversion of N-acetyl-L-glutamic acid to N-acetyl-L-glutamic acid semialdehyde; conversion of N-acetyl-L-glutamic acid semialdehyde to N- acetyl-L-ornithine; conversion of N-acetyl-L-ornithine to L-ornithine; conversion of L-ornithine to L-citrulline; conversion of L-citrulline to arginino-succinic acid; conversion of arginino-succinic acid to L-arginine; conversion of L-arginine to guanidinoacetic acid; and, finally, conversion of guanidinoacetic acid to creatine. Fan Wenchao proposes, that the microorganism overexpresses one or more enzymes selected from the group consisting of N-acetylglutamate-synthase, N-acetylornithine-δ-aminotransferase, N-acetylornithinase, ornithine-carbamoyl transferase, argininosuccinate synthetase, glycine amidino-transferase (EC: 2.1. 4.1), and guanidinoacetate N-methyltransferase (EC: 2.1.1.2). The microorganism overexpresses preferably glycine aminotransferase (L-arginine:glycine amidinotransferase) and guanidinoacetate N-methyltransferase.

Until now, microorganisms suitable for an increased production of GAA compared to their wildtype forms and a respective method for the production of GAA using such microorganisms have not been reported.

Therefore, the problem underlying the present invention is to provide a microorganism transformed to be capable of producing guanidinoacetic acid (GAA) and to a method for the fermentative production of GAA using such microorganism.

The problem is solved by a microorganism comprising an increased activity by overexpression of an enzyme having the function of a carbamoylphosphate synthase (EC 6.3.4.16) compared to the respective enzymic activity in the wildtype microorganism and comprising at least one heterologous gene coding for a protein having the function of an L-arginine:glycine amidinotransferase (AGAT).

A heterologous gene means that the gene has been inserted into a host organism which does not naturally have this gene. Insertion of the heterologous gene in the host is performed by recombinant DNA technology. Microorganisms that have undergone recombinant DNA technology are called transgenic, genetically modified or recombinant. Thus, the microorganism according to the present invention is recombinant.

The increased activity of the enzyme having the function of a carbamoylphosphate synthase is achieved by overexpression of a gene coding for the enzyme having the function of a carbamoylphosphate synthase.

The activity of the L-arginine:glycine amidinotransferase may also be increased by overexpression of the gene coding for the L-arginine:glycine amidinotransferase.

Proteins having the function of an L-arginine:glycine amidinotransferase (AGAT) belong to the amidinotransferase family. The amidinotransferase family comprises glycine (EC:2.1.4.1) and inosamine (EC:2.1 .4.2) amidinotransferases, enzymes involved in creatine and streptomycin biosynthesis respectively. This family also includes arginine deiminases, EC:3.5.3.6. These enzymes catalyse the reaction: arginine + $H_2O$ <=> citrulline + $NH_3$. Also found in this family is the Streptococcus anti tumour glycoprotein. Enzymes or proteins with an L-arginie:glycine-amidinotransferase (AGAT) activity are also described to possess a conserved domain that belongs to the PFAM Family: Amidinotransf (PF02274) (: Marchler-BauerA et al. (2017), "CDD/SPARCLE: functional classification of proteins via subfamily domain architectures.", Nucleic Acids Res. 45(D1):D200-D203.) as described also in the following publications: Pissowotzki K et al., Mol Gen Genet 1991;231:113-123 (PUBMED:1661369 EPMC:1661369); D'Hooghe I et al., J Bacteriol 1997;179:7403-7409 (PUBMED:9393705 EPMC:9393705); Kanaoka M et al. , Jpn J Cancer Res 1987;78:1409-1414 (PUBMED:3123442 EPMC:3123442). Particular examples of AGATs are those of *Moorea producens, Homo sapiens, Rattus norvegicus, Galeopterus variegatus,* and of *Cylindrospermopsis raciborskii.*

The microorganism according to the present invention ideally has an improved ability to produce L-arginine compared with the ability of the wildtype microorganism. This property may be achieved by selection of microorganisms that are natural L-arginine producers or may have acquired the ability to produce L-arginine by mutation.

The microorganism according to the present invention and having an improved ability to produce L-arginine compared with the ability of the wildtype microorganism may have an increased activity of an enzyme having the function of an argininosuccinate lyase (E.C. 4.3.2.1) compared to the respective enzymic activity in the wildtype microorganism.

Furthermore, in the microorganism according to the present invention, the activity of an enzyme having the function of an ornithine carbamoyltransferase (EC 2.1.3.3) may be increased compared to the respective enzymic activity in the wildtype microorganism.

In the microorganism according to the present invention, the activity of an enzyme having the function of an argininosuccinate synthetase (E.C. 6.3.4.5) may be also increased compared to the respective enzymic activity in the wildtype microorganism.

Increased enzyme activities in microorganisms can be achieved, for example, by mutation of the corresponding endogenous gene. A further measure to increase enzymic activities may be to stabilize the mRNA coding for the enzymes.

The increased activities of the above-mentioned enzymes may also be achieved by overexpressing the genes coding for the respective enzymes. In other words, the problem is preferably solved by a microorganism having an improved ability to produce L-arginine compared with the ability of the wildtype organism and/or having at least one or more overexpressed genes (e.g. carA, carB) coding for a protein having the function of a carbamoylphosphate synthase (EC 6.3.4.16), and further comprising a gene coding for a protein having the function of an L-arginine:glycine amidinotransferase (AGAT, e.g. EC 2.1.4.1).

The microorganism according to the present invention preferably also comprises at least one or more overexpressed genes selected from the group consisting of a gene (e.g. *argF*/*argF2*/*argI*) coding for a protein having the function of an ornithine carbamoyltransferase (EC 2.1.3.3), a gene (e.g. *argG*) coding for a protein having the function of an argininosuccinate synthetase (E.C. 6.3.4.5), and a gene (e.g. *argH*) coding for a protein having the function of an argininosuccinate lyase (E.C. 4.3.2.1).

Overexpression of a gene is generally achieved by increasing the copy number of the gene and/or by functionally linking the gene with a strong promoter and/or by enhancing the ribosomal binding site and/or by codon usage optimization of the start codon or of the whole gene or a combination comprising a selection of all methods mentioned above.

In the context of the present invention, a microorganism having an improved ability to produce L-arginine means a microorganism producing L-arginine in excess of its own need. Examples for such L-arginine producing microorganisms are e.g. *C. glutamicum* ATCC 21831 or those disclosed by Park et al. (NATURE COMMUNICATIONS | DOI: 10.1038/ncomms5618) or by Ginesy et al. (Microbial Cell Factories (2015) 14:29).

In one embodiment of the microorganism according to the present invention the arginine operon (*argCJBDFR*) may be overexpressed.

Alternatively, in the microorganism according to the present invention the *argR* gene coding for the arginine responsive repressor protein ArgR may be attenuated or deleted.

In a further embodiment of the present invention and, optionally in addition to the above-mentioned modifications, at least one or more of the genes coding for an enzyme of the biosynthetic pathway of L-arginine, comprising of *gdh, argJ, argB, argC* and/or *argD* coding for a glutamate dehydrogenase, an ornithine acetyltransferase, an acetylglutamate kinase, an acetylglutamylphosphate reductase and an acetylornithine aminotransferase, respectively, is overexpressed in the microorganism according to the present invention.

Table 1 shows the different names of enzymes involved in or contributing to arginine biosynthesis in different species, i.e. *E. coli, C. glutamicum* and *Pseudomonas putida* (*P. putida*).

In the microorganism of the present invention the gene coding for a protein having the function of an L-arginine:glycine amidinotransferase may further be overexpressed. Overexpression of a gene is generally achieved by increasing the copy number of the gene and/or by functionally linking the gene with a strong promoter and/or by enhancing the ribosomal binding site and/or by codon usage optimization of the start codon of the whole gene or a combination comprising a selection or all methods mentioned above.

Table 1: Names of Enzymes

| Enzyme name | Alias | EC Number | *E. coli* | *C. glutamicum* | *P. putida* |
|---|---|---|---|---|---|
| glutamate dehydrogenase | NADP-SPECIFIC GLUTAMATE DEHYDROGENASE | 1.4.1.4 (1.4.1.2 in P. putida) | gdhA | gdh | gdhA/ gdhB |
| N-acetyl glutamokinase | ACETYLGLUTAMATE KINASE | 2.7.2.8 | argB | argB | argB |

(continued)

| Enzyme name | Alias | EC Number | E. coli | C. glutamicum | P. putida |
|---|---|---|---|---|---|
| N-acetylglutamyl phosphate reductase | N-acetyl-gamma-glutamylphosphate reductase | 1.2.1.38 | argC | argC | argC |
| N-acetylglutamic acid-γ-semialdehyde dehydrogenase | ACETYLORNITHINE AMINOTRANSFERASE (in e.c. bifunctional acetylornithine aminotransferase/ succinyldiaminopimelate aminotransferase) | 2.6.1.11 / 2.6.1.17 | argD | argD | aruC |
| acetylornithine deacetylase | bifunctional acetylornithine deacetylase / GLUTAMATE N-ACETYLTRANSFERASE | 2.3.1.35 / 2.3.1.1 | | argJ | argJ |
| acetylornithine deacetylase | | 3.5.1.16 | argE | | argE |
| | GLUTAMATE N-ACETYLTRANSFERASE | 2.3.1.1 | argA | | argA |
| ornithine carbamoyltransferase | ornithine carbamoyltransferase 1 | 2.1.3.3 | argI | argF/argF2 | arcB/ argF |
| Argininosuccinate synthetase | ARGININOSUCCINATE SYNTHASE | 6.3.4.5 | argG | argG | argG |
| Argininosuccinate lyase | ARGININOSUCCINATE LYASE | 4.3.2.1 | argH | argH | argH |
| carbamoyl-phosphate synthase | carbamoyl-phosphate synthase | 6.3.5.5 | carAB | carAB | carAB |
| carbamate kinase | carbamate kinase | 2.7.2.2 | ybcF /yqeA | | arcC |

The protein having the function of an L-arginine:glycine amidinotransferase (AGAT) in the microorganism of the present invention may comprise an amino acid sequence which is at least 70 % homologous, preferably at least 80 % or at least 90 % homologous to the amino acid sequence according to SEQ ID NO: 2 or to SEQ ID NO: 4 ("AGAT_Mp" of *Moorea producens*). In a further embodiment of the present invention the amino acid sequence of the L-arginine:glycine amidinotransferase is identical to amino acid sequence according to SEQ ID NO: 2 or to SEQ ID NO: 4 (cf. Database UniProt, 15 February 2017, "Glycine amidinotransferase", XP055706853, EBI accession no. UNIPROT: A0A1D8TKD3). The sequence of wildtype DNA coding for the *Moorea producens* AGAT is SEQ ID NO: 1, the corresponding DNA sequence that has been codon optimized for *C. glutamicum* is SEQ ID NO: 3. The corresponding DNA sequence for the *Moorea producens* AGAT gene that has been codon optimized for P. putida is SEQ ID NO: 33.

The protein having the function of an L-arginine:glycine amidinotransferase in the microorganism of the present invention may comprise an amino acid sequence which is at least 70 % homologous, preferably at least 80 % or at least 90 % homologous to the amino acid sequence according to SEQ ID NO:16 or to SEQ ID NO:26 ("AGAT_cyrA" of *Cylindrospermopsis raciborskii* ATW205). In a further embodiment of the present invention the amino acid sequence of the L-arginine:glycine amidinotransferase is identical to amino acid sequence according to SEQ ID NO: 16 or to SEQ ID NO:26. The sequence of wildtype DNA coding for the *Cylindrospermopsis raciborskii* AGAT is SEQ ID NO: 15, the corresponding DNA sequence that has been codon optimized for *C. glutamicum* is SEQ ID NO: 25.

The protein having the function of an L-arginine:glycine amidinotransferase in the microorganism of the present invention may comprise an amino acid sequence which is at least 70 % homologous, preferably at least 80 % or at least 90 % homologous to the amino acid sequence according to SEQ ID NO: 23 ("AGAT_Gv" of *Galeopterus variegatus*), preferably to SEQ ID NO: 24 or to SEQ ID NO:32. In a further embodiment of the present invention the amino acid sequence of the L-arginine:glycine amidinotransferase is identical to amino acid sequence according to SEQ ID NO: 24 or to SEQ ID NO:32 The sequence of the corresponding *Galeopterus variegatus* AGAT DNA that has been codon optimized for *C. glutamicum* is SEQ ID NO: 31.

The protein having the function of an L-arginine:glycine amidinotransferase in the microorganism of the present invention may comprise an amino acid sequence which is at least 70 % homologous, preferably at least 80 % or at least 90 % homologous to the amino acid sequence according to SEQ ID NO:18 ("AGAT_Hs" of *homo sapiens*), preferably to SEQ ID NO: 20 or to SEQ ID NO:28, e.g. the amino acid sequence according to SEQ ID NO: 21 or to SEQ ID NO: 22 or to SEQ ID NO:30 (each "AGAT Rn" of *Rattus norvegicus*; the corresponding DNA, codon optimized for C. *glutamicum* is SEQ ID NO: 29). In a further embodiment of the present invention the amino acid sequence of the L-arginine:glycine amidinotransferase is identical to amino acid sequence according to SEQ ID NO: 18. The sequence of wildtype DNA coding for the *Homo sapiens* AGAT is SEQ ID NO: 17, the corresponding DNA sequence that has been codon optimized for C. *glutamicum* is SEQ ID NO: 27.

The microorganism of the present invention may belong to the genus *Corynebacterium,* preferably *Corynebacterium glutamicum* (*C. glutamicum*), or to the genus *Enterobacteriaceae,* preferably *Escherichia coli* (*E. coli*), or to the genus *Pseudomonas,* preferably *Pseudomonas putida* (*P. putida*).

Generally, increased enzyme activities in microorganisms can be achieved, for example, by mutation of the corresponding endogenous gene. Enzyme activities can also be enhanced by overexpression of the corresponding gene.

Generally, the overexpression of a gene, according to the present invention, is achieved by increasing the copy number of the gene and/or by an enhancement of regulatory factors, e.g. by functionally linking the gene with a strong promoter and/or by enhancing the ribosomal binding site and/or by codon usage optimization of the start codon or of the whole gene. The enhancement of such regulatory factors which positively influence gene expression can, for example, be achieved by modifying the promoter sequence upstream of the structural gene in order to increase the effectiveness of the promoter or by completely replacing said promoter with a more effective or a so-called strong promoter. Promoters are located upstream of the gene. A promoter is a DNA sequence consisting of about 40 to 50 base pairs and which constitutes the binding site for an RNA polymerase holoenzyme and the transcriptional start point, whereby the strength of expression of the controlled polynucleotide or gene can be influenced. Generally, it is possible to achieve an overexpression or an increase in the expression of genes in bacteria by selecting strong promoters, for example by replacing the original promoter with strong, native (originally assigned to other genes) promoters or by modifying certain regions of a given, native promoter (for example its so-called -10 and -35 regions) towards a consensus sequence, e.g. as taught by M. Patek et al. (Microbial Biotechnology 6 (2013), 103-117) for *C. glutamicum*. A "functional linkage" is understood to mean the sequential arrangement of a promoter with a gene, which leads to a transcription of the gene.

The genetic code is degenerated which means that a certain amino acid may be encoded by a number of different triplets. The term codon usage refers to the observation that a certain organism will typically not use every possible codon for a certain amino acid with the same frequency. Instead an organism will typically show certain preferences for specific codons meaning that these codons are found more frequently in the coding sequence of transcribed genes of an organism. If a certain gene foreign to its future host, i.e. from a different species, should be expressed in the future host organism the coding sequence of said gene should then be adjusted to the codon usage of said future host organism (i.e. codon usage optimization).

The above-mentioned problem is further solved by a method for the fermentative production of guanidino acetic acid (GAA), comprising the steps of a) cultivating the microorganism according to the present invention as defined above in a suitable medium under suitable conditions, and b) accumulating GAA in the medium to form an GAA containing fermentation broth.

The method according to the present invention may further comprise adding glycine and/or adding L-arginine and/or adding L-ornithine to the medium. Preferably, the medium is supplemented with glycine in a concentration ranging from 0.1 to 300 g glycine/l medium, preferably 0.82 g glycine/l medium, and/or with L-arginine to obtain a concentration ranging from 0.1 to 200 g L-arginine/l medium, preferably 1.9 g L-arginine/l medium.

The method of the present invention may further comprise the step of isolating GAA from the fermentation broth.

The method according to the present invention may further comprise the step of drying and/or granulating the GAA containing fermentation broth.

The present invention further concerns a microorganism as defined above, further comprising a gene coding for an enzyme having the activity of a guanidinoacetate N-methyltransferase (EC: 2.1.1.2). Preferably, the gene coding for an enzyme having the activity of a guanidinoacetate N-methyltransferase is overexpressed.

The present invention also concerns a method for the fermentative production of creatine, comprising the steps of a) cultivating the microorganism according to the present invention comprising a gene coding for an enzyme having the activity of a guanidinoacetate N-methyltransferase in a suitable medium under suitable conditions, and b) accumulating creatine in the medium to form a creatine containing fermentation broth.

Preferably, the method further comprises isolating creatine from the creatine containing fermentation broth. Creatine may be extracted from fermentation broth by isoelectric point method and / or ion exchange method. Alternatively, creatine can be further purified by a method of recrystallization in water.

**EXPERIMENTAL SECTION**

**A) MATERIALS and METHODS**

**Chemicals**

Kanamycin solution from *Streptomyces kanamyceticus* was purchased from Sigma Aldrich (St. Louis, USA, Cat. no. K0254). IPTG (Isopropyl β-D-1-thiogalactopyranoside) was purchased from Carl-Roth (Karlsruhe, Germany, Cat. no. 2316.4.). If not stated otherwise, all other chemicals were purchased analytically pure from Merck (Darmstadt, Germany), Sigma Aldrich (St. Louis, USA) or Carl-Roth (Karlsruhe, Germany).

**Cultivation for cell proliferation**

If not stated otherwise, cultivation / incubation procedures were performed as follows herewith:

a. LB broth (MILLER) from Merck (Darmstadt, Germany; Cat. no. 110285) was used to cultivate *E. coli* strains in liquid medium. The liquid cultures (10 ml liquid medium per 100 ml Erlenmeyer flask with 3 baffles) were incubated in the Infors HT Multitron standard incubator shaker from Infors GmbH (Bottmingen, Switzerland) at 30°C and 200 rpm.
b. LB agar (MILLER) from Merck (Darmstadt, Germany, Cat. no. 110283) was used for cultivation of *E. coli* strains on agar plates. The agar plates were incubated at 30°C in an INCU-Line® mini incubator from VWR (Radnor, USA).
c. Brain heart infusion broth (BHI) from Merck (Darmstadt, Germany, Cat. no. 110493) was used to cultivate *C. glutamicum* strains in liquid medium. The liquid cultures (10 ml liquid medium per 100 ml Erlenmeyer flask with 3 baffles) were incubated in the Infors HT Multitron standard incubator shaker from Infors GmbH (Bottmingen, Switzerland) at 30°C and 200 rpm.
d. Brain heart agar (BHI-agar) from Merck (Darmstadt, Germany, Cat. no. 113825) was used for cultivation of *C. glutamicum* strains on agar plates. The agar plates were incubated at 30°C in an incubator from Heraeus Instruments with Kelvitron® temperature controller (Hanau, Germany).
e. For cultivating *C. glutamicum* after electroporation, BHI-agar (Merck, Darmstadt, Germany, Cat. no. 113825) was supplemented with 134 g/l sorbitol (Carl Roth GmbH + Co. KG, Karlsruhe, Germany), 2.5 g/l yeast extract (Oxoid/ThermoFisher Scientific, Waltham, USA, Cat. no. LP0021) and 25 mg/l kanamycin. The agar plates were incubated at 30°C in an incubator from Heraeus Instruments with Kelvitron® temperature controller (Hanau, Germany).

**Determining optical density of bacterial suspensions**

a. The optical density of bacterial suspensions in shake flask cultures was determined at 600 nm (OD600) using the BioPhotometer from Eppendorf AG (Hamburg, Germany).
b. The optical density of bacterial suspensions produced in the Wouter Duetz (WDS) micro fermentation system (24-Well Plates) was determined at 660 nm (OD660) with the GENios™ plate reader from Tecan Group AG (Männedorf, Switzerland).

**Centrifugation**

a. Bacterial suspensions with a maximum volume of 2 ml were centrifuged in 1.5 ml or 2 ml reaction tubes (e.g. Eppendorf Tubes® 3810X) using an Eppendorf 5417 R benchtop centrifuge (5 min. at 13.000 rpm).
b. Bacterial suspensions with a maximum volume of 50 ml were centrifuged in 15 ml or 50 ml centrifuge tubes (e.g. FalconTM 50 ml Conical Centrifuge Tubes) using an Eppendorf 5810 R benchtop centrifuge for 10 min. at 4.000 rpm.

**DNA isolation**

Plasmid DNA was isolated from E. *coli* cells using the QIAprep Spin Miniprep Kit from Qiagen (Hilden, Germany, Cat. No. 27106) according to the instructions of the manufacturer.

**Polymerase chain reaction (PCR)**

PCR with a proof reading (high fidelity) polymerase was used to amplify a desired segment of DNA for Sanger sequencing or DNA assembly. Non-proof-reading polymerase Kits were used for determining the presence or absence of a desired DNA fragment directly from *E. coli* or *C. glutamicum* colonies.
a. The Phusion® High-Fidelity DNA Polymerase Kit (Phusion Kit) from New England BioLabs Inc. (Ipswich, USA, Cat. No. M0530) was used for template-correct amplification of selected DNA regions according to the instructions of the manufacturer (see table 2).

Table 2: Thermocycling conditions for PCR with Phusion® High-Fidelity DNA Polymerase Kit from New England BioLabs Inc.

| PCR Program | | | |
|---|---|---|---|
| Step | Time [min.:sec.] | T [°C] | Description |
| 1 | 00:30 | 98 | Initial denaturation step |
| 2 | 00:05 | 98 | Denaturation step |
| 3 | 00:30 | 60 | Annealing step |
| 4 | 00:xx | 72 | Elongation step 1 min. per kb DNA |
| | | | Repeat step 2 to 4: 35 x |
| 5 | 05:00 | 72 | Final elongation step |
| 6 | Hold | 4 | Cooling step |

b. Taq PCR Core Kit (Taq Kit) from Qiagen (Hilden, Germany, Cat. No.201203) was used to amplify a desired segment of DNA in order to confirm its presence. The kit was used according to the instructions of the manufacturer (see table 3).

Table 3: Thermocycling conditions for PCR with Taq PCR Core Kit (Taq Kit) from Qiagen.

| PCR Program | | | |
|---|---|---|---|
| Step | Time [min.:sec.] | T [°C] | Description |
| 1 | 05:00 | 94 | Initial denaturation step |
| 2 | 00:30 | 94 | Denaturation step |
| 3 | 00:30 | 52 | Annealing step |
| 4 | 01:20 | 72 | Elongation step 1 min. per kb DNA |
| | | | Repeat step 2 to 4: 35 x |
| 5 | 04:00 | 72 | Final elongation step |
| 6 | Hold | 4 | Cooling step |

c. SapphireAmp® Fast PCR Master Mix (Sapphire Mix) from Takara Bio Inc (Takara Bio Europe S.A.S., Saint-Germain-en-Laye, France, Cat. No. RR350A/B) was used as an alternative to confirm the presence of a desired segment of DNA in cells taken from *E. coli* or *C. glutamicum* colonies according to the instructions of the manufacturer (see table 4).

Table 4: Thermocycling conditions for PCR with SapphireAmp® Fast PCR Master Mix (Sapphire Mix) from Takara Bio Inc.

| PCR Program | | | |
|---|---|---|---|
| Step | Time [min.:sec.] | T [°C] | Description |
| 1 | 01:00 | 94 | Initial denaturation step |

(continued)

| PCR Program | | | |
|---|---|---|---|
| Step | Time [min.:sec.] | T [°C] | Description |
| 2 | 00:05 | 98 | Denaturation step |
| 3 | 00:05 | 55 | Annealing step |
| 4 | 00:05 | 72 | Elongation step |
| | | | Repeat step 2 to 4: 30 x |
| 5 | 04:00 | 72 | Final elongation step |
| 6 | Hold | 4 | Cooling step |

d. All oligonucleotide primers were synthesized by Eurofins Genomics GmbH (Ebersberg, Germany) using the phosphoramidite method described by McBride and Caruthers (1983).

e. As PCR template either a suitably diluted solution of isolated plasmid DNA or of total DNA isolated from a liquid culture or the total DNA contained in a bacterial colony (colony PCR) was used. For said colony PCR the template was prepared by taking cell material with a toothpick from a colony on an agar plate and placing the cell material directly into the PCR reaction tube. The cell material was heated for 10 sec. with 800 W in a microwave oven type Mikrowave & Grill from SEVERIN Elektrogeräte GmbH (Sundern, Germany) and then the PCR reagents were added to the template in the PCR reaction tube.

f. All PCR reactions were carried out in PCR cyclers type Mastercycler or Mastercycler nexus gradient from Eppendorf AG (Hamburg, Germany).

**Restriction enzyme digestion of DNA**

For restriction enzyme digestions either "FastDigest restriction endonucleases (FD)" (ThermoFisher Scientific, Waltham, USA) or restriciton endonucleases from New England BioLabs Inc. (Ipswich, USA) were used. The reactions were carried out according to the instructions of the manufacturer's manual.

**Determining the sizes of DNA fragments**

a. The sizes of small DNA fragments (<1000 bps) were usually determined by automatic capillary electrophoresis using the QIAxcel from Qiagen (Hilden, Germany).

b. If DNA fragments needed to be isolated or if the DNA fragments were >1000 bps DNA was separated by TAE agarose gel electrophoresis and stained with GelRed® Nucleic Acid Gel Stain (Biotium, Inc., Fremont, Canada). Stained DNA was visualized at 302 nm.

**Purification of PCR amplificates and restriction fragments**

PCR amplificates and restriction fragments were cleaned up using the QIAquick PCR Purification Kit from Qiagen (Hilden, Germany; Cat. No. 28106), according to the manufacturer's instructions. DNA was eluted with 30 μl 10 mM Tris*HCl (pH 8.5).

**Determining DNA concentration**

DNA concentration was measured using the NanoDrop Spectrophotometer ND-1000 from PEQLAB Biotechnologie GmbH, since 2015 VWR brand (Erlangen, Germany).

**Assembly cloning**

Plasmid vectors were assembled using the "NEBuilder HiFi DNA Assembly Cloning Kit" purchased from New England BioLabs Inc. (Ipswich, USA, Cat. No. E5520). The reaction mix, containing the linear vector and at least one DNA insert, was incubated at 50°C for 60 min.. 0.5 μl of Assembly mixture was used for each transformation experiment.

**Chemical transformation of E. *coli***

For plasmid cloning, chemically competent "NEB® Stable Competent E. *coli* (High Efficiency)" (New England BioLabs Inc., Ipswich, USA, Cat. No. C3040) were transformed according to the manufacturer's protocol. Successfully transformed cells were selected on LB agar supplemented with 25 mg/l kanamycin.

**Transformation of *C. glutamicum***

Transformation of *C. glutamicum* with plasmid-DNA was conducted via electroporation using a "Gene Pulser Xcell" (Bio-Rad Laboratories GmbH, Feldkirchen, Germany) as described by Ruan *et al.* (2015). Electroporation was performed in 1 mm electroporation cuvettes (Bio-Rad Laboratories GmbH, Feldkirchen, Germany) at 1.8 kV and a fixed time constant set to 5 ms. Transformed cells were selected on BHI-agar containing 134 g/l sorbitol, 2.5 g/l Yeast Extract and 25 mg/l kanamycin.

**Determining nucleotide sequences**

Nucleotide sequences of DNA molecules were determined by Eurofins Genomics GmbH (Ebersberg, Germany) by cycle sequencing, using the dideoxy chain termination method of Sanger et al. (Proceedings of the National Academy of Sciences USA 74, 5463 - 5467, 1977), on Applied Biosystems® (Carlsbad, CA, USA) 3730xl DNA Analyzers. Clonemanager Professional 9 software from Scientific & Educational Software (Denver, USA) was used to visualise and evaluate the sequences.

**Glycerol stocks of E. *coli* and *C. glutamicum* strains**

For long time storage of E. *coli*- and *C. glutamicum* strains glycerol stocks were prepared. Selected E. *coli* clones were cultivated in 10 ml LB medium supplemented with 2 g/l glucose. Selected *C. glutamicum* clones were cultivated in 10 ml twofold concentrated BHI medium supplemented with 2 g/l glucose. Cultures of plasmid containing E. *coli*- and *C. glutamicum* strains were supplemented with 25 mg/l kanamycin. The medium was contained in 100 ml Erlenmeyer flasks with 3 baffles. It was inoculated with a loop of cells taken from a colony. The culture was then incubated for 18 h at 30°C and 200 rpm. After said incubation period 1.2 ml 85 % (v/v) sterile glycerol were added to the culture. The obtained glycerol containing cell suspension was then aliquoted in 2 ml portions and stored at -80°C.

**GAA production in millilitre-scale cultivations**

The millilitre-scale cultivation system according to Duetz (2007) was used to assess the GAA-production of the strains. For this purpose, 24-deepwell microplates (24 well WDS plates) from EnzyScreen BV (Heemstede, Netherlands, Cat. no. CR1424) filled with 2.5 ml medium per well were used.

Precultures of the strains were done in 10 ml seed medium (SM). The medium was contained in a 100 ml Erlenmeyer flask with 3 baffles. It was inoculated with 100 $\mu$l of a glycerol stock culture and the culture incubated for 24 h at 30°C and 200 rpm. The composition of the seed medium (SM) is shown in table 5.

Table 5: Seed medium (SM)

| Components | Concentration (g/l) |
|---|---|
| Yeast extract FM902 (Angel Yeast Co.,LTD, Hubei, P.R.China) | 10 |
| Urea | 1.5 |
| $KH_2PO_4$ | 0.5 |
| $K_2HPO_4$ | 0.5 |
| $MgSO_4 * 7 H_2O$ | 1 |
| Biotin | 0.0001 |
| Thiamine hydrochloride | 0.0001 |
| $FeSO_4 * 7 H_2O$ | 0.01 |
| $MnSO_4 * H_2O$ | 0.01 |

(continued)

| Components | Concentration (g/l) |
|---|---|
| Glucose | 20 |
| pH=7.0 | |

After said incubation period the optical densities OD600 of the precultures were determined. The volume, needed to inoculate 2.5 ml of production medium (PM) to an OD600 of 0.1, was sampled from the preculture, centrifuged (1 min at 8000 g) and the supernatant was discarded. Cells were then resuspended in 100 $\mu$l of production medium.

The main cultures were started by inoculating the 2.4 ml production medium (PM) containing wells of the 24 Well WDS-Plate with each 100 $\mu$l of the resuspended cells from the precultures. The composition of the production medium (PM) is shown in table 6.

Table 6: Production medium (PM)

| Components | Concentration (g/l) |
|---|---|
| 3-(N-morpholino)propanesulfonic acid (MOPS) | 40 |
| Yeast extract FM902 (Angel Yeast Co.,LTD, Hubei, P.R.China) | 1.5 |
| $(NH_4)_2SO_4$ | 10 |
| $NH_4Cl$ | 15 |
| Trisodium citrate * 2 $H_2O$ | 10 |
| Urea | 1 |
| $KH_2PO_4$ | 0.5 |
| $K_2HPO_4$ | 0.5 |
| $MgSO_4$ * 7 $H_2O$ | 1 |
| Biotin | 0.0001 |
| Thiamine hydrochloride | 0.0001 |
| $FeSO_4$ * 7 $H_2O$ | 0.01 |
| $MnSO_4$ * $H_2O$ | 0.01 |
| $ZnSO_4$ * 7 $H_2O$ | 0.000015 |
| $CuSO_4$ * 5 $H_2O$ | 0.0004 |
| Antifoam XFO-1501 (Ivanhoe Industries Inc., Zion, USA) | 0.5 |
| Glucose | 40 |
| IPTG (Isopropyl $\beta$-D-1-thiogalactopyranoside) | 0.3 mM |
| pH=7.2 | |

The main cultures were incubated for 72 h at 30 °C and 300 rpm in an Infors HT Multitron standard incubator shaker from Infors GmbH (Bottmingen, Switzerland) until complete consumption of glucose. The glucose concentration in the suspension was analysed with the blood glucose-meter OneTouch Vita® from LifeScan (Johnson & Johnson Medical GmbH, Neuss, Germany).

After cultivation the culture suspensions were transferred to a deep well microplate. A part of the culture suspension was suitably diluted to measure the OD600. Another part of the culture was centrifuged and the concentration of GAA in the supernatant was analyzed as described below.

**Determination of L-arginine and glycine content in yeast pepton FM902**

As yeast extract FM902 (Angel Yeast Co.,LTD, Hubei, P.R.China) contains various peptides and amino acids, its content of L-arginine and glycine was measured as follows.

For measuring free amino acids, the samples were prepared by dissolving 1 g of yeast extract in 20 ml of water. The solution was filled up with water to a total volume of 25 ml, mixed thoroughly and filtered using a 0.2 $\mu$M nylon syringe filter.

For measuring total amino acids (free amino acids plus amino acids bound in peptides), the samples were prepared by dissolving 1 g yeast extract in 10 ml 6M HCl and incubating them for 24h at 110°C. Then, water was added up to a total volume of 25 ml. The solution was mixed thoroughly and filtered using a 0.2 $\mu$M nylon syringe filter.

The concentrations of L-arginine and glycine in the samples were determined by ion exchange chromatography using a SYKAM S433 amino acid analyzer from SYKAM Vertriebs GmbH (Fürstenfeldbruck, Germany). As solid phase a column with spherical, polystyrene-based cation exchanger (Peek LCA N04/Na, dimension 150 x 4.6 mm) from SYKAM was used. Depending on the L-amino acid the separation takes place in an isocratic run using a mixture of buffers A and B for elution or by gradient elution using said buffers. As buffer A an aqueous solution containing in 20 1263 g trisodium citrate, 120 g citric acid, 1100 ml methanol, 100 ml 37 % HCl and 2 ml octanoic acid (final pH 3.5) was used. As buffer B an aqueous solution containing in 20 1392 g trisodium citrate, 100 g boric acid and 2 ml octanoic acid (final pH 10.2) was used. The free amino acids were coloured with ninhydrin through post-column derivatization and detected photometrically at 570 nm.

Table 7 shows the content of free and total L-arginine and glycine determined in yeast extract FM902 (Angel Yeast Co.,LTD, Hubei, P.R.China), as well as the resulting amounts in the production medium (PM).

Table 7: Content of L-arginine and glycine in yeast extract (YE) FM902 and resulting concentrations in production medium (PM) containing 1.5 g/l YE.

| Amino acid | Free amino acid in YE | Total amino acid in YE | Resulting free amino acid in PM | Resulting total amino acid in PM |
|---|---|---|---|---|
| L-arginine | 15.1 g/kg | 32.1 g/kg | 22.7 mg/l | 48.2 mg/l |
| glycine | 6.9 g/kg | 30.5 g/kg | 10.4 mg/l | 45.7 mg/l |

**Quantification of GAA**

Samples were analyzed with an analyzing system from Agilent, consisting of a HPLC "Infinity 1260" coupled with a mass analyzer "Triple Quad 6420" (Agilent Technologies Inc., Santa Clara, USA). Chromatographic separation was done on the Atlantis HILIC Silica column, 4,6X250mm, 5$\mu$m (Waters Corporation, Milford, USA) at 35°C. Mobile phase A was water with 10mM ammonium formate and 0,2% formic acid. Mobile phase B was a mixture of 90% acetonitrile and 10 % water, 10 mM ammonium formate were added to the mixture. The HPLC system was started with 100% B, followed by a linear gradient for 22 min and a constant flow rate of 0,6 mL/min to 66% B. The mass analyzer was operated in the ESI positive ionization mode. For detection of GAA the m/z values were monitored by using an MRM fragmentation [M+H] + 118 - 76. The limit of quantification (LOQ) for GAA was fixed to 7 ppm.

**B) EXPERIMENTAL RESULTS**

**Example 1: Synthesis of genes coding for L-arginine:glycine amidinotransferases (AGAT, EC 2.1.4.1) from various organisms**

*Moorea producens* is a filamentous cyanobacterium. The genome of the *Moorea producens* strain PAL-8-15-08-1 was published By Leao et al. (Leao T, Castelão G, Korobeynikov A, Monroe EA, Podell S, Glukhov E, Allen EE, Gerwick WH, Gerwick L, Proc Natl Acad Sci USA. 2017 Mar 21;114(12):3198-3203. doi: 10.1073/pnas.1618556114; accession number CP017599.1). It contains an open reading frame putatively coding for a L-arginine:glycine amidinotransferase (AGAT, EC 2.1.4.1, accession number BJP34_00300, SEQ ID NO:1). SEQ ID NO:2 and SEQ ID NO: 4 show the derived amino acid sequence (accession number WP_070390602) which was designated as AGAT_Mp.

The gene cyrA from Cylindrospermopsis raciborskii AWT205 (accession number EU140798.1) codes for a L-arginine:glycine amidinotransferase (Mihali TK, Kellmann R, Muenchhoff J, Barrow KD, Neilan BA (2008) "Characterization of the gene cluster responsible for cylindrospermopsin biosynthesis.", Appl Environ Microbiol., 74(3):716-22, doi: 10.1128/AEM.01988-07; SEQ ID NO:15). SEQ ID NO:16 and SEQ ID NO:26 show the derived amino acid sequence (accession number ABX60160) which was designated as AGAT_cyrA.

The cDNA sequence of the human L-arginine:glycine amidinotransferase was described by Humm *et al.,* 1994 (Humm A, Huber R, Mann K (1994) "The amino acid sequences of human and pig I-arginine:glycine amidinotransferase.", FEBS Letters, Vol. 339 (1-2), 101-107, DOI: 10.1016/0014-5793(94)80394-3; accession number NM_001482.3, SEQ ID NO:17). The derived amino acid sequence (accession number NP_001473.1, SEQ ID NO:18) starts with a mitochondrial transit peptide (amino acids 1 - 37) which is absent in the mature enzyme. A truncated enzyme, starting at amino acid 56, was found to be active when expressed in *E. coli* (Humm A, Fritsche E, Mann K, Göhl M, Huber R (1997) "Recombinant expression and isolation of human L-arginine : glycine amidinotransferase and identification of its active-site cysteine residue." Biochem. J. 322, 771-776, DOI: 10.1042/bj3220771). An N-terminal fusion of a 7 amino acid tag (SEQ ID NO:19) was shown to improve protein expression in *E. coli* (Hansted JG, Pietikäinen L, Hög F, Sperling-Petersen HU, Mortensen KK (2011) "Expressivity tag: A novel tool for increased expression in Escherichia coli." Journal of Biotechnology 155 (2011) 275- 283, DOI:0.1016/j.jbiotec.2011.07.013). Accordingly a fusion protein consisting of the tag and the truncated AGAT was designed and it was designated as AGAT_Hs (SEQ ID NO:20 and SEQ ID NO:28).

The amino acid sequence of the L-arginine:glycine amidinotransferase from *Rattus norvegicus* (accession number NP_112293.1, SEQ ID NO:21) is highly similar to the human enzyme. As described for the human enzyme, the sequence was used to design a fusion protein consisting of the N-terminal expression tag and the truncated sequence of the enzyme. The resulting fusion protein was designated as AGAT_Rn (SEQ ID NO:22 and SEQ ID NO:30).

The sunda flying lemur *Galeopterus variegatus* has a predicted L-arginine:glycine amidinotransferase (accession number NP_112293.1, , SEQ ID NO:23). As described for the human enzyme, its amino acid sequence was used to design a fusion protein consisting of the N-terminal expression tag and the truncated sequence of the enzyme. The resulting fusion protein was designated as AGAT_Gv (SEQ ID NO:24 and SEQ ID NO:32).

Using the software tool "GeneOptimizer" (Geneart/ ThermoFisher Scientific, Waltham, USA) the amino acid sequences of AGAT_Mp, AGAT_cyrA, AGAT_Hs, AGAT_Rn and AGAT_Gvwere translated back into DNA sequences and optimized for the codon usage of *C. glutamicum.* Their ends were expanded with sequences for assembly cloning and Shine-Dalgarno-Sequences were added upstream of the open reading frames. The resulting DNA sequences are SEQ ID NO:3 (coding for AGAT_Mp), SEQ ID NO:25 (coding for AGAT_cyrA), SEQ ID NO:27 (coding for AGAT_Hs), SEQ ID NO:29 (coding forAGAT_Rn) and SEQ ID NO:31 (coding for AGAT_Gv). They were ordered for gene synthesis from Invitrogen/Geneart (Thermo Fisher Scientific, Waltham, USA). The synthetic genes were delivered as parts of cloning plasmids which were designated as pMA-T_AGAT_Mp, pMA-T_AGAT_cyrA, pMA-T_AGAT_Hs, pMA-T_AGAT_Rn and pMA-T_AGAT_Gv.

**Example 2: Cloning of AGAT_Mp into the expression plasmid pEC-XK99E**

The *E. coli-C. glutamicum* shuttle plasmid pEC-XK99E was digested using the restriction endonuclease Smal. Terminal phosphates were removed using the "FastAP Thermosensitive Alkaline Phosphatase" (Thermo Fisher Scientific, Waltham, USA). The DNA was then purified with the "QIAquick PCR Purification Kit" (Qiagen GmbH, Hilden, Germany).

The cloning plasmid pMA-T AGAT_Mp was digested with Mlul + Aatll and the resulting fragments were blunted using the "Fast DNA End Repair Kit" (Thermo Fisher Scientific, Waltham, USA). They were separated by agarose gel electrophoresis (0,8% agarose in TAE buffer) and the band corresponding to "AGAT_Mp" (1174 bp) was cut out. Its DNA was purified using the "QIAquick Gel Extraction Kit" (Qiagen GmbH, Hilden, Germany).

The AGAT_Mp fragment and the linearized pEC-XK99E were ligated using the "Ready-To-Go T4 DNA ligase" (GE Healthcare Europe GmbH, Freiburg, Germany). The ligation product was transformed into "NEB Stable Competent *E. coli* (High Efficiency)" (New England Biolabs, Ipswich, USA) and the cells were grown on LB agar containing 25 mg/l kanamycin. Appropriate clones were identified by restriction enzyme digestion and DNA sequencing. The resulting plasmid was named pEC-XK99E_AGAT_Mp.

**Example 2.1: Cloning of AGAT_Mp and AGAT_cyrA into the expression plasmid pEKEx2**

The *E. coli-C. glutamicum* shuttle plasmid pEKEx2 (Eikmanns, 1991) was digested using the restriction endonuclease Pstl. The resulting fragments were blunted using the "Fast DNA End Repair Kit" (Thermo Fisher Scientific) and terminal phosphates were removed with "FastAP Thermosensitive Alkaline Phosphatase" (Thermo Fisher Scientific, Waltham, USA). The DNA was then purified with the "QIAquick PCR Purification Kit" (Qiagen GmbH, Hilden, Germany).

The cloning plasmids pMA-T_AGAT_Mp and pMA-T_AGAT_cyrA were digested with Mlul + Aatll and the resulting fragments were blunted using the "Fast DNA End Repair Kit" (Thermo Fisher Scientific, Waltham, USA). They were separated by agarose gel electrophoresis (0,8% agarose in TAE buffer) and the bands corresponding to AGAT_Mp (1174 bp) and AGAT_cyrA (1204 bp) were cut out. The DNA was purified using the "QIAquick Gel Extraction Kit" (Qiagen GmbH, Hilden, Germany).

Each AGAT fragment was ligated with the linearized pEKEx2 using the "Ready-To-Go T4 DNA ligase" (GE Healthcare Europe GmbH, Freiburg, Germany). The ligation products were transformed into "NEB Stable Competent *E. coli* (High Efficiency)" (New England Biolabs, Ipswich, USA) and the cells were grown on LB agar containing 25 mg/l kanamycin. Appropriate clones were identified by restriction enzyme digestion and DNA sequencing. The resulting plasmids were named pEKEx2_AGAT_Mp and pEKEx2_AGAT_cyrA.

**Example 2.2: Cloning of AGAT_Hs, AGAT_Rn and AGAT_Gv into the expression plasmid pEKEx2**

The cloning plasmids pMA-T_AGAT_Hs, pMA-T_AGAT_Rn and pMA-T_AGAT_Gv were digested with Eco31I and the products were purified using the "QIAquick Gel Extraction Kit" (Qiagen GmbH, Hilden, Germany).

The *E. coli-C. glutamicum* shuttle plasmid pEKEx2 (Eikmanns BJ, Kleinertz E, Liebl W, Sahm H (1991) "A family of Corynebacterium glutamicum/Escherichia coli shuttle vectors for cloning, controlled gene expression, and promoter probing.", Gene. 1991 Jun 15;102(1):93-8) was digested using the restriction endonucleases SbfI and BamHI. The DNA was purified with the "QIAquick PCR Purification Kit" (Qiagen GmbH, Hilden, Germany).

Each of the AGAT fragments was assembled with the digested pEKEx2 using the "NEBuilder HiFi DNA Assembly Cloning Kit" (New England BioLabs Inc., Ipswich, USA, Cat. No. E5520). The assembly products were transformed into "NEB Stable Competent *E. coli* (High Efficiency)" (New England Biolabs, Ipswich, USA) and the cells were grown on LB agar containing 25 mg/l kanamycin. Appropriate clones were identified by restriction enzyme digestion and DNA sequencing. The resulting plasmids were designated as pEKEx2_AGAT_Hs, pEKEx2_AGAT_Rn and pEKEx2_AGAT_Gv respectively.

**Example 3: Cloning of the gene *argF* into plasmid pCR-Blunt II-TOPO**

The *argF* gene was PCR amplified with the Phusion High-Fidelity DNA Polymerase Kit (New England BioLabs Inc., Ipswich, USA) using genomic DNA of *C. glutamicum* ATCC13032 and the oligonucleotide primer argF_1.p (SEQ ID NO:5) and argF_2.p (SEQ ID NO:6). The resulting PCR product was cloned into the plasmid pCR-Blunt **II-TOPO** (Thermo Fisher Scientific/Invitrogen, Waltham, USA) and a proper plasmid clone was identified by restriction enzyme digestion and DNA sequencing. This plasmid was named **pCRII-argF.**

**Example 4: Cloning of the genes *argG* and *argH* into plasmid pCR-Blunt II-TOPO**

The genes *argG* and *argH* were PCR amplified with the Phusion High-Fidelity DNA Polymerase Kit (New England BioLabs Inc., Ipswich, USA) using genomic DNA of *C. glutamicum* ATCC13032 and the oligonucleotide primer argG_1.p (SEQ ID NO:7) and argH_2.p (SEQ ID NO:8). The resulting PCR product was cloned into the plasmid pCR-Blunt II-TOPO (Thermo Fisher Scientific/Invitrogen, Waltham, USA) and a proper plasmid clone was identified by restriction enzyme digestion and DNA sequencing. The plasmid was named pCRII-argGH.

**Example 5: Cloning of the genes *argG* and *argH* into plasmid pCRII-argF**

pCRII-argGH was cut using HpaI + AvrII and a restriction fragment of 2773 bps was isolated from an agarose gel. pCRII-argF was cut using SspI + AvrII and a restriction fragment of 4526 bps was isolated from an agarose gel. Both fragments were ligated and then transformed into *E. coli.* Proper plasmid clones were identified by restriction enzyme digestion and DNA sequencing. The resulting plasmid was named pCRII-argFGH.

**Example 6: Cloning of the genes *argF, argG* and *argH* into the expression plasmid pEC-XK99E**

pCRII-argFGH was cut using HpaI + AvrII and a restriction fragment of 2773 bps was isolated from an agarose gel. The plasmid pEC-XK99E was cut using Ecl136II + XbaI. A restriction fragment of 6999 bps was isolated from an agarose gel. Both fragments were ligated and then transformed into *E. coli.* Proper plasmid clones were identified by restriction digestion and DNA sequencing. The resulting plasmid pEC-XK99E_argFGH contains the genes *argF, argG* and *argH* from *C. glutamicum.*

**Example 7: Cloning of the genes *argF, argG* and *argH* into the expression plasmid pEC-XK99E_AGAT_Mp**

pCRII-argFGH was cut using XbaI + SpeI and a restriction fragment of 3868 bps was isolated from an agarose gel. The plasmid pEC-XK99E AGAT_Mp was cut using XbaI. A restriction fragment of 8188 bps was isolated from an agarose gel. Both fragments were ligated and then transformed into *E. coli.* Proper plasmid clones were identified by restriction digestion and DNA sequencing. The resulting plasmid pEC-XK99E_AGAT_Mp_argFGH contains the genes *argF, argG* and *argH* from *C. glutamicum* in combination with AGAT_Mp.

### Example 8: Cloning of the gene *argF into* the expression plasmid pEC-XK99E_AGAT_Mp

pCRII-argF was cut using KpnI + XbaI + AseI and the DNA was purified with the "QIAquick PCR Purification Kit" (Qiagen GmbH, Hilden, Germany). The plasmid pEC-XK99E_AGAT_Mp was cut using KpnI + XbaI, the DNA was purified with the "QIAquick PCR Purification Kit" (Qiagen GmbH, Hilden, Germany). Both eluates were mixed, the DNA fragments were ligated and the product was used to transform *E. coli.* Proper plasmid clones were identified by restriction digestion and DNA sequencing. The resulting plasmid pEC-XK99E_AGAT_Mp_argF contains the gene *argF from C. glutamicum* in combination with AGAT_Mp.

### Example 9: Cloning of the gene *argG* into the expression plasmid pEC-XK99E_AGAT_Mp

pCRII-argGH was cut using XbaI + SalI and a restriction fragment of 1798 bps was isolated from an agarose gel. The plasmid pEC-XK99E_AGAT_Mp was cut using XbaI + SalI, the DNA was purified with the "QIAquick PCR Purification Kit" (Qiagen GmbH, Hilden, Germany). The DNA fragments were ligated and the product was used to transform *E. coli.* Proper plasmid clones were identified by restriction digestion and DNA sequencing. The resulting plasmid pEC-XK99E_AGAT_Mp_argG contains the gene *argG* from *C. glutamicum* in combination with AGAT_Mp.

### Example 10: Chromosomal insertion of the sod promoter upstream of the *carAB* operon in ATCC13032

The enzymatic activity of the carbamoyl phosphate synthetase was increased by genomic insertion of the strong *sod*-promoter upstream of the *carAB* operon in ATCC13032. Therefore, the plasmid pK18mobsacB_Psod-carAB was constructed as follows. Plasmid pK18mobsacB was cut using EcoRI + HindIII and the linearized vector DNA (5670 bps) was cut out of an agarose gel. The DNA was extracted using the "QIAquick Gel Extraction Kit" (QIAGEN GmbH, Hilden, Germany).

For constructing the insert, three DNA fragments were created by PCR with the following pairs of primers (genomic DNA of ATCC13032 as template):

PsodcarAB-LA-F (SEQ ID NO:9) + PsodcarAB-LA-R (SEQ ID NO:10)
= left homology arm (1025 bps)
PsodcarAB-F (SEQ ID NO:11) + PsodcarAB-R (SEQ ID NO:12)
= sod-promoter (250 bps)
PsodcarAB-RA-F SEQ ID NO:13) + PsodcarAB-RA-R (SEQ ID NO:14)
= right homology arm (944 bps)

The product DNAs were purified using the "QIAquick PCR Purification Kit" (Qiagen GmbH, Hilden, Germany). The linearized plasmid and the PCR products were then assembled using the "NEBuilder HiFi DNA Assembly Cloning Kit" (New England BioLabs Inc., Ipswich, USA, Cat. No. E5520). Proper plasmid clones were identified by restriction digestion and DNA sequencing.

pK18mobsacB_Psod-carAB was used to integrate the strong sod-promoter upstream of the *carAB* genes into the genome of *C. glutamicum* ATCC13032. The plasmid was transformed into ATCC13032 by electroporation. Chromosomal integration (resulting from a first recombination event) was selected by plating on BHI agar supplemented with 134 g/l sorbitol, 2.5 g/l yeast extract and 25 mg/l kanamycin. The agar plates were incubated for 48 h at 33°C.

Individual colonies were transferred onto fresh agar plates (with 25 mg/l kanamycin) and incubated for 24 h at 33°C. Liquid cultures of these clones were cultivated for 24 h at 33°C in 10 ml BHI medium contained in 100 ml Erlenmeyer flasks with 3 baffles. To isolate clones that have encountered a second recombination event, an aliquot was taken from each liquid culture, suitably diluted and plated (typically 100 to 200 $\mu$l) on BHI agar supplemented with 10 % saccharose. These agar plates were incubated for 48 h at 33°C. The colonies growing on the saccharose containing agar plates were then examined for kanamycin sensitivity. To do so a toothpick was used to remove cell material from the colony and to transfer it onto BHI agar containing 25 mg/l kanamycin and onto BHI agar containing 10 % saccharose. The agar plates were incubated for 60 h at 33°C. Clones that proved to be sensitive to kanamycin and resistant to saccharose were examined by PCR and DNA sequencing for the appropriate integration of the sod promoter. The resulting strain was named ATCC13032_Psod-carAB.

Table 8: List of strains

| Strain | Comment |
|---|---|
| **Escherichia coli** | |
| NEB® Stable | Commercial cloning strain (New England BioLabs Inc., Ipswich, USA) |
| MG1655 | *E. coli* reference strain (Blattner *et al.,* 1997*)) |
| **Corynebacterium glutamicum** | |
| ATCC13032 | *Corynebacterium glutamicum* wild type strain (Kinoshita *et al.,* 1957**)) |
| ATCC21831 | *C. glutamicum* variant producing L-arginine (Park *et al.,* 2014***)) |
| ATCC13032_P*sod-carAB* | Chromosomal integration of the strong *sod* promotor upstream of *carAB* in *C. glutamicum* ATCC13032 |
| *) Blattner et al., 1997: Blattner FR, Plunkett G 3rd, Bloch CA, Perna NT, Burland V, Riley M, Collado-Vides J, Glasner JD, Rode CK, Mayhew GF, Gregor J, Davis NW, Kirkpatrick HA, Goeden MA, Rose DJ, Mau B, Shao Y, Science. 1997 Sep 5;277(5331):1453-62. (doi: 10.1126/science.277.5331.1453)<br>**) Kinoshita S, Udaka S, Shimono M., J. Gen. Appl. Microbiol. 1957; 3(3): 193-205.<br>***) Park et al., 2014: Park SH, Kim HU, Kim TY, Park JS, Kim SS, Lee, Nat Commun. 2014 Aug 5; 5:4618. (doi: 10.1038/ncomms5618) | |

Table 9: List of plasmids

| Plasmid | Comment |
|---|---|
| pMA-T_AGAT_Mp | Gene synthesis and cloning vector for AGAT_Mp (*Moorea producens)*; proprietary plasmid of Invitrogen/Geneart (Thermo Fisher Scientific, Waltham, USA) |
| pMA-T_AGAT_cyrA | Gene synthesis and cloning vector for AGAT_cyrA (*Cylindrospermopsis raciborskii* AWT205); proprietary plasmid of Invitrogen/Geneart (Thermo Fisher Scientific, Waltham, USA) |
| pMA-T_AGAT_Hs | Gene synthesis and cloning vector for AGAT_Hs (*Homo sapiens*); proprietary plasmid of Invitrogen/Geneart (Thermo Fisher Scientific, Waltham, USA) |
| pMA-T_AGAT_Rn | Gene synthesis and cloning vector for AGAT_Rn (*Rattus norvegicus*); proprietary plasmid of Invitrogen/Geneart (Thermo Fisher Scientific, Waltham, USA) |
| pMA-T_AGAT_Gv | Gene synthesis and cloning vector for AGAT_Gv (*Galeopterus variegatus*); proprietary plasmid of Invitrogen/Geneart (Thermo Fisher Scientific, Waltham, USA) |
| pEKEx2 | Empty *E. coli - C. glutamicum* shuttle plasmid |
| pEKEx2_AGAT_Mp | Expression of AGAT_Mp *(Moorea producens)* |
| pEKEx2_AGAT_cyrA | Expression of AGAT_cyrA (*Cylindrospermopsis raciborskii* AWT205) |
| pEKEx2_AGAT_Hs | Expression of AGAT_Hs (*Homo sapiens*) |
| pEKEx2_AGAT_Rn | Expression of AGAT_Rn (*Rattus norvegicus*) |
| pEKEx2_AGAT_Gv | Expression of AGAT_Gv (*Galeopterus variegatus*) |
| pEC-XK99E | Empty *E. coli - C. glutamicum* shuttle plasmid |
| pEC-XK99E_AGAT_Mp | Expression of AGAT_Mp *(Moorea producens)* |
| pCR-Blunt II-TOPO | Empty plasmid for cloning PCR products (Thermo Fisher Scientific/Invitrogen, Waltham, USA) |
| pCRII-argF | pCR-Blunt II-TOPO containing the cloned gene *argF* (*C. glutamicum*) |
| pCRII-argGH | pCR-Blunt II-TOPO containing the cloned genes *argG, argH* (*C. glutamicum*) |
| pCRII-argFGH | pCR-Blunt II-TOPO containing the cloned genes *argF, argG, argH* (*C. glutamicum*) |

(continued)

| Plasmid | Comment |
|---|---|
| pEC-XK99E_argFGH | Expression of *argF, argG, argH* (*C. glutamicum*) |
| pEC-XK99E_AGAT_Mp_argFGH | Expression of AGAT_Mp (*Moorea producens*), *argF, argG, argH* (*C. glutamicum*) |
| pEC-XK99E_AGAT_Mp_argGH | Expression of AGAT_Mp (*Moorea producens*), *argG, argH* (*C. glutamicum*) |
| pEC-XK99E_AGAT_Mp_argF | Expression of AGAT_Mp (*Moorea producens*), *argF* (*C. glutamicum*) |
| pEC-XK99E_AGAT_Mp_argG | Expression of AGAT_Mp (*Moorea producens*), *argG* (*C. glutamicum*) |
| pK18mobsacB | Empty plasmid for genome modification of *C. glutamicum* |
| pK18mobsacB_Psod-carAB | Plasmid for chromosomal integration of the strong sod promotor upstream of *carAB* in *C. glutamicum* |

**Example 11: Transformation of C. *glutamicum* strains with various expression plasmids**

The following strains of *C. glutamicum* were transformed with various plasmids:

- *C. glutamicum* ATCC13032 (Kinoshita et al., J. Gen. Appl. Microbiol. 1957; 3(3): 193-205) is a commonly used wild type strain.
- *C. glutamicum* strain ATCC21831 (Park et al., Nat Commun. 2014 Aug 5; 5:4618) synthesizes L-arginine from primary substrates like ammonia and glucose.
- ATCC13032_P*sod-carAB* is a variant of ATCC13032 with the strong sod promotor upstream of the *carAB* genes.

The strains were transformed by electroporating with various plasmids (as shown in table 10). Plasmid containing cells were selected with 25 mg/l kanamycin.

Table 10: List of plasmid-containing *C. glutamicum* strains

| Plasmid | Cloned gene | Recipient strain | Resulting strain |
|---|---|---|---|
| pEKEx2 | none | ATCC21831 | ATCC21831/pEKEx2 |
| pEKEx2_AGAT_Mp | AGAT_Mp | ATCC21831 | ATCC21831/pEKEx2_AGAT_Mp |
| pEKEx2_AGAT_Hs | AGAT_Hs | ATCC21831 | ATCC21831/pEKEx2_AGAT_Hs |
| pEKEx2_AGAT_Rn | AGAT_Rn | ATCC21831 | ATCC21831/pEKEx2_AGAT_Rn |
| pEKEx2_AGAT_Gv | AGAT_Gv | ATCC21831 | ATCC21831/pEKEx2_AGAT_Gv |
| pEKEx2_AGAT_cyrA | AGAT_cyrA | ATCC21831 | ATCC21831/pEKEx2_AGAT_cyr A |
| pEC-XK99E | none | ATCC13032 | ATCC13032/pEC-XK99E |
| pEC-XK99E_argFGH | *argF, argG, argH* | ATCC13032 | ATCC13032/pEC-XK99E_argFGH |
| pEC-XK99E_AGAT_Mp | AGAT_Mp | ATCC13032 | ATCC13032/pEC-XK99E_AGAT_Mp |
| pEC-XK99E_AGAT_Mp_a rgG | AGAT_Mp, *argG* | ATCC13032 | ATCC13032/pEC-XK99E_AGAT_Mp_argG |
| pEC-XK99E_AGAT_Mp_a rgF | AGAT_Mp, *argF* | ATCC13032 | ATCC13032/pEC-XK99E_AGAT_Mp_argF |
| pEC-XK99E_AGAT_Mp_a rgGH | AGAT_Mp, *argG,* argH | ATCC13032 | ATCC13032/pEC-XK99E_AGAT_Mp_argGH |

(continued)

| Plasmid | Cloned gene | Recipient strain | Resulting strain |
|---|---|---|---|
| pEC-XK99E_AGAT_Mp_a rgFGH | AGAT_Mp, *argF, argG, argH* | ATCC13032 | ATCC13032/pEC-XK99E_AGAT_Mp_argFGH |
| pEC-XK99E | none | ATCC13032_Psod-carAB | ATCC13032_Psod-carAB/pEC-XK99E |
| pEC-XK99E_AGAT_Mp_a rgFGH | AGAT_Mp, argF, argG, argH | ATCC13032_Psod-carAB | ATCC13032_Psod-carAB/pEC-XK99E_AGAT_Mp_argFGH |

### Example 12: Impact of AGAT and substrate availability on GAA production

Strains ATCC13032/pEC-XK99E_AGAT_Mp (carrying a gene for the AGAT enzyme from *Moorea producens*) and ATCC13032/pEC-XK99E (empty vector for control) were analyzed for their ability to produce GAA in batch cultivations using the system of Wouter Duetz (as described above). The production medium (PM) contained 40 g/l D-glucose as the main carbon source. Some batches were supplemented with L-arginine and/or glycine as indicated.

Table 11: GAA production by strains ATCC13032/pEC-XK99E and ATCC13032/pEC-XK99E_AGAT_Mp

| Strain | Supplementation | GAA |
|---|---|---|
| ATCC13032/pEC-XK99E | none | not detectable |
| ATCC13032/pEC-XK99E | 1.90 g/l Arg and 0.818 g/l Gly | not detectable |
| ATCC13032/pEC-XK99E_AGAT_Mp | none | 25 mg/l |
| ATCC13032/pEC-XK99E_AGAT_Mp | 0.818 g/l Gly | 31 mg/l |
| ATCC13032/pEC-XK99E_AGAT_Mp | 1.90 g/l Arg and 0.818 g/l Gly | 124 mg/l |

As shown in table 11, the control strain ATCC13032/pEC-XK99E could not produce GAA, even if the precursors L-arginine and glycine were provided. We conclude that it does not possess an intrinsic AGAT activity. Strain ATCC13032/pEC-XK99E_AGAT_Mp contains a polynucleotide coding for a putative AGAT from *Moorea producens*. It produced 25 mg/l GAA in unsupplemented PM. Supplementation of glycine resulted in a small increase to 31 mg/l GAA. Supplementation of glycine and L-arginine largely increased GAA production to 124 mg/l.

### Example 13: Production of GAA from primary substrates

In an industrial GAA production process, supplementation of L-arginine would be rather costly when compared to primary substrates like ammonia, urea and glucose. It would therefore be desirable to generate GAA directly from such primary substrates.

For this purpose, the L-arginine producer *C. glutamicum* ATCC21831 was transformed with

- pEKEx2 (empty vector for control),
- pEKEx2_AGAT_Mp (containing the AGAT_Mp gene from *Morea producens*),
- pEKEx2_AGAT_Hs (containing the AGAT_Hs gene from *Homo sapiens*),
- pEKEx2_AGAT_Rn (containing the AGAT_Rn gene from *Rattus norvegicus*),
- pEKEx2_AGAT_Gv (containing the AGAT_Gv gene from *Galeopterus variegatus*) and
- pEKEx2_AGAT_cyrA (containing the AGAT_cyrA gene from *Cylindrospermopsis raciborskii*).

ATCC21831 was isolated as a canavanine resistant mutant and it was found to produce L-arginine. Its genome was sequenced by Park et al. (Nat Commun. 2014 Aug 5;5:4618. doi: 10.1038/ncomms5618; accession number CP007722) and the strain is publicly available from LGC Standards (LGC Standards GmbH, Wesel, Germany).

All transformed ATCC21831 strains were analyzed for their ability to produce GAA in batch cultivations using the system of Wouter Duetz (as described above). The production medium (PM) contained 40 g/l D-glucose as the main carbon source. Some batches were supplemented with glycine and/or L-arginine.

Table 12: GAA production by ATCC21831 strains transformed with pEKEx2 vector containing AGAT from different species in CGAF and MOPS medium with 1.5 g/l yeast extract

| Strain | Supplementation | GAA |
|---|---|---|
| ATCC21831/pEKEx2 | none | not detectable |
| ATCC21831/pEKEx2 | 1.90 g/l arginine, 0.82 g/l glycine | not detectable |
| ATCC21831/pEKEx2_AGAT_Mp | none | 26.5 mg/l |
| ATCC21831/pEKEx2_AGAT_Mp | 0.82 g/l glycine | 166.3 mg/l |
| ATCC21831/pEKEx2_AGAT_Hs | none | 3.6 mg/l |
| ATCC21831/pEKEx2_AGAT_Hs | 0.82 g/l glycine | 56.2 mg/l |
| ATCC21831/pEKEx2_AGAT_Rn | none | 0.9 mg/l |
| ATCC21831/pEKEx2_AGAT_Rn | 0.82 g/l glycine | 7.6 mg/l |
| ATCC21831/pEKEx2_AGAT_Gv | none | 0.7 mg/l |
| ATCC21831/pEKEx2_AGAT_Gv | 0.82 g/l glycine | 8.5 mg/l |
| ATCC21831/pEKEx2_AGAT_cyrA | none | 20.8 mg/l |
| ATCC21831/pEKEx2_AGAT_cyrA | 0.82 g/l glycine | 52.7 mg/l |

As shown in table 12, ATCC21831/pEKEx2 did not produce GAA, even if the precursors L-arginine and glycine were present. We conclude that ATCC21831/ pEKEx2 does not have an intrinsic AGAT activity. The transformed strains ATCC21831/ pEKEx2_AGAT_Mp, ATCC21831 ZpEKEx2_AGAT_Hs, ATCC21831 ZpEKEx2_AGAT_Rn, ATCC21831/pEKEx2_AGAT_Gv, and ATCC21831/pEKEx2_AGAT_cyrA produced from about 1 up to 26 mg/l GAA in unsupplemented PM. We conclude that the precursors L-arginine and glycine were synthesized from the primary substrates D-glucose, ammonium and urea.

When glycine was added, GAA production of ATCC21831/pEKEx2_AGAT_Mp, ATCC21831/pEKEx2_AGAT_Hs,ATCC21831/pEKEx2_AGAT_Rn, ATCC21831/pEKEx2_AGAT_Gv, and ATCC21831/pEKEx2_AGAT_cyrA significantly increased compared to the non-supplemented experiments. When compared to strain ATCC13032/pEC-XK99E_AGAT_Mp (see table 10), the L-arginine producer ATCC21831 having an AGAT gene accumulates much more GAA when glycine is not limiting. We conclude that the capability of internally providing L-arginine improves GAA production.

**Example 14: Impact of L-arginine regeneration on GAA production**

In L-arginine production strains (e.g. ATCC21831) the intermediate L-ornithine is synthesized de *novo* and further converted into L-arginine. When such a strain is provided with an AGAT, the enzyme will produce an equal molar amount of GAA and L-ornithine. The formation of L-ornithine though consumes a significant amount of the primary C- and N-sources and it thus lowers the yield of GAA.

We found that enhancement of the biosynthetic pathway leading from L-ornithine to L-arginine improves GAA production, presumably by improving the recycling of L-ornithine to L-arginine.

Various strains, derived from ATCC13032, were cultivated using the Wouter Duetz system and then analyzed for their ability to produce GAA (tables 13, 14 and 15).

Table 13: Impact of improved L-arginine regeneration on GAA production in PM comprising YE without glycine or L-arginine supplementation

| Strain | GAA |
|---|---|
| ATCC13032/pEC-XK99E | 0 mg/l |
| ATCC13032/pEC-XK99E_argFGH | 0 mg/l |
| ATCC13032_Psod-carAB/pEC-XK99E | 0 mg/l |
| ATCC13032/pEC-XK99E_AGAT_Mp | 25 mg/l |
| ATCC13032/pEC-XK99E_AGAT_Mp_argG | 26 mg/l |

(continued)

| Strain | GAA |
|--------|-----|
| ATCC13032/pEC-XK99E_AGAT_Mp_argH | 24 mg/l |
| ATCC13032/pEC-XK99E_AGAT_Mp_argF | 27 mg/l |
| ATCC13032/pEC-XK99E_AGAT_Mp_argGH | 26 mg/l |
| ATCC13032/pEC-XK99E_AGAT_Mp_argFGH | 25 mg/l |
| ATCC13032_Psod-carAB/pEC-XK99E_AGAT_Mp | 43 mg/l |
| ATCC13032_Psod-carAB/pEC-XK99E_AGAT_Mp_argG | 45 mg/l |
| ATCC13032_Psod-carAB/pEC-XK99E_AGAT_Mp_ argFGH | 45 mg/l |

Table 14: Impact of improved L-arginine regeneration on GAA production in PM without YE. All cultures were supplemented with 0.82 g/l glycine

| Strain | GAA |
|--------|-----|
| ATCC13032/pEC-XK99E | 0 mg/l |
| ATCC13032/pEC-XK99E_argFGH | 0 mg/l |
| ATCC13032_Psod-carAB/pEC-XK99E | 0 mg/l |
| ATCC13032/pEC-XK99E_AGAT_Mp | 22 mg/l |
| ATCC13032/pEC-XK99E_AGAT_Mp_argG | 24 mg/l |
| ATCC13032/pEC-XK99E_AGAT_Mp_argH | 22 mg/l |
| ATCC13032/pEC-XK99E_AGAT_Mp_argF | 23 mg/l |
| ATCC13032/pEC-XK99E_AGAT_Mp_argGH | 23 mg/l |
| ATCC13032/pEC-XK99E_AGAT_Mp_argFGH | 23 mg/l |
| ATCC13032_Psod-carAB/pEC-XK99E_AGAT_Mp | 53 mg/l |

Table 15: Impact of improved L-arginine regeneration on GAA production in PM comprising YE. All cultures were supplemented with 0.82 g/l glycine and 1.9 g/l L-arginine.

| Strain | GAA |
|--------|-----|
| ATCC13032/pEC-XK99E | not detectable |
| ATCC13032/pEC-XK99E_argFGH | not detectable |
| ATCC13032_Psod-carAB/pEC-XK99E | not detectable |
| ATCC13032/pEC-XK99E_AGAT_Mp | 124 mg/l |
| ATCC13032/pEC-XK99E_AGAT_Mp_argG | 136 mg/l |
| ATCC13032/pEC-XK99E_AGAT_Mp_argF | 136 mg/l |
| ATCC13032/pEC-XK99E_AGAT_Mp_argGH | 133 mg/l |
| ATCC13032/pEC-XK99E_AGAT_Mp_argFGH | 154 mg/l |
| ATCC13032_Psod-carAB/pEC-XK99E_AGAT_Mp | 156 mg/l |
| ATCC13032_Psod-carAB/pEC-XK99E_AGAT_Mp_argFGH | 171 mg/l |

As shown in tables 13 to 15, strains lacking the AGAT gene did not produce a detectable amount of GAA.
The expression of AGAT_Mp in ATCC13032/pEC-XK99E_AGAT_Mp resulted in 124 mg/l GAA. Additional amplification of *argG* (strain ATCC13032/pEC-XK99E_AGAT_Mp_argG), *argF* (strain ATCC13032/pEC-XK99E_AGAT_Mp_argF) or *argG+argH* (strain ATCC13032/pEC-XK99E_AGAT_Mp_argGH) improved the production of GAA (cf. table 14).

In strain ATCC13032/pEC-XK99E_AGAT_Mp_argFGH the expression of the genes *argF* (coding for ornithine carbamoyltransferase), *argG* (coding for argininosuccinate synthetase) and *argH* (coding for argininosuccinate lyase) is enhanced. This further improved the production of GAA to 154 mg/l (cf. table 14).

The conversion of L-ornithine to L-citrulline, catalyzed by the ornithine carbamoyltransferase, depends on the availability of the co-substrate carbamoyl phosphate. Carbamoyl phosphate is produced by the carbamoyl phosphate synthase, which is encoded by the genes *carA* and *carB*. In strain ATCC13032_Psod-carAB/pEC-XK99E AGAT_Mp a genomic insertion of the strong sod-promoter enhances the expression of *carA* and *carB*. When compared to ATCC13032/pEC-XK99E_AGAT_Mp, this resulted in improved GAA production (156 mg/l vs. 124 mg/l).

In strain ATCC13032_Psod-carAB/pEC-XK99E_AGAT_Mp_argFGH the improved L-ornithine conversion (overexpression of *argF, argG* and *argH*) was combined with the improved carbamoyl phosphate biosynthesis (overexpression of *carA* and *carB*). This combination further improved GAA production to 171 mg/l.

## Example 15: Construction of a *P. putida* expression vector for the *Moorea producens* gene AGAT_Mp

For the heterologous expression of the AGAT from *Moorea producens* (EC 2.1.4.1, SEQ ID NO:2 and SEQ ID NO:4) in *P. putida* KT2440 the plasmid pACYCATh-5{PRha}[agat_Mp(coPp)] was constructed. A codon optimized AGAT_Mp gene was to be cloned under the control of the rhamnose inducible promoter $P_{rha}$ into the vector pACYATh-5. Downstream of the AGAT_Mp gene a terminator sequence is located. The AGAT_Mp gene was ordered for gene synthesis from Eurofins Genomics Germany GmbH (Ebersberg, Germany) and the DNA sequence of the gene fragment was codon-optimized for expression in *P. putida* KT2440 (SEQ ID NO:33). Upstream of the open reading frame a Shine-Dalgarno sequence was added. The $P_{Rha}$ promoter cassette (SEQ ID NO:34) and the terminator sequence (SEQ ID NO:35) were amplified from *E. coli* K12 genomic DNA. The vector is based on pACYC184 (New England BioLabs Inc., Ipswich, USA) and carries a p15A origin of replication for *E. coli* and a pVS1 origin of replication for the replication in *P. putida* KT2440. The pVS1 origin comes from the *Pseudomonas* plasmid pVS1 (Itoh Y, Watson JM, Haas D, Leisinger T, Plasmid 1984, 11(3), 206-20). In the next step the AGAT_Mp gene fragment was amplified via PCR using the primer MW_20_01_fw (SEQ ID NO:36) and MW_20_02_rv (SEQ ID NO:37) and cloned into the vector pACYCATh-5 using the restriction sites *Apa*I/*Xho*I and NEBuilder® HiFi DNA Assembly Cloning Kit from (New England BioLabs Inc., Ipswich, USA, Cat. No. E5520). The assembled product was transformed into 10-beta electrocompetent *E. coli* cells (New England BioLabs Inc., Ipswich, USA, Cat. No. C3020K). Procedure of PCR purification, cloning and transformation were carried out according to manufacturer's manual. The correct insertion of the target gene was checked by restriction analysis and the authenticity of the introduced DNA fragments was verified by DNA sequencing. The resulting expression vector was named pACYCATh-5{PRha}[agat_Mp(coPp)] (SEQ ID NO:38, see table 17).

The *P. putida* strain KT2440 was transformed with the plasmid pACYCATh-5{PRha}[agat_Mp(coPp)] by means of electroporation and plated onto LB-agar plates supplemented with tetracycline (10 mg/l). Transformants were checked for the presence of the correct plasmid by plasmid preparation and analytic restriction analysis. The resulting strain was named *P. putida* KT2440/pACYCATh-5{PRha}[agat_Mp(coPp)] (see table 18).

## Example 16: Construction of a *P. putida* expression vector for the *Moorea producens* gene AGAT_Mp and *P. putida* genes *argF, argG* and *argH*

For the heterologous expression of AGAT_Mp from *Moorea producens* and *argF* (SEQ ID NO:39), *argG* (SEQ ID NO:41), *argH* (SEQ ID NO:43) from *P. putida* KT2440 the plasmid pACYCATh-5{PRha}[agat_Mp(coPp) argFGH_Pp] was constructed. The synthetic operon consisting of AGAT_Mp encoding an L-arginine:glycine amidinotransferase (AGAT, EC 2.1.4.1, SEQ ID NO:2 and SEQ ID NO:4), argF encoding an ornithine carbamoyltransferase (ArgF, EC 2.1.3.3, SEQ ID NO:40), *argG* encoding an argininosuccinate synthase (ArgG, E.C. 6.3.4.5, SEQ ID NO:42) and *argH* encoding an argininosuccinate lyase (ArgH, E.C. 4.3.2.1, SEQ ID NO:44), respectively, was cloned under the control of the rhamnose inducible promoter $P_{rha}$ into the vector pACYCATh-5. Downstream of the synthetic operon a terminator sequence is located. The AGAT_Mp gene was ordered for gene synthesis from Eurofins Genomics Germany GmbH (Ebersberg, Germany) and the DNA sequence of the gene fragment was codon-optimized for expression in *P. putida* KT2440. The genes *argFGH* were also synthesized as gene fragment *argFGH* (SEQ ID NO: 45). The $P_{Rha}$ promoter cassette (SEQ ID NO:34) and the terminator sequence (SEQ ID NO:35) were amplified from *E. coli* K12 genomic DNA. The vector is based on pACYC184 (New England BioLabs Inc., Ipswich, USA) and carries a p15A origin of replication for *E. coli* and a pVS1 origin of replication for the replication in *P. putida* KT2440. The pVS1 origin comes from the *Pseudomonas* plasmid pVS1 (Itoh Y, Watson JM, Haas D, Leisinger T, Plasmid 1984, 11(3), 206-20). For cloning AGAT_Mp and *argFGH* were amplified via PCR. Primers used for cloning are listed in table 16. The PCR products were cloned into the vector pACYCATh-5 using the restriction sites *Apal/Xhol* and NEBuilder® HiFi DNA Assembly Cloning Kit from (New England BioLabs Inc., Ipswich, USA, Cat. No. E5520) to generate an optimized operon. For amplification the Phusion™ High-Fidelity Master Mix from New England Biolabs (Ipswich, USA) was used according to manufacturer's manual. The assembled product was transformed into 10-beta electrocompetent *E. coli* cells (New England BioLabs Inc., Ipswich, USA, Cat. No. C3020K). Procedure of PCR purification, cloning and transformation was carried out according to manufacturer's manual. The correct insertion of the target genes was checked by restriction analysis and the authenticity of the introduced DNA fragments was verified by DNA sequencing. The resulting expression vector was named pACYCATh-5{PRha}[agat_Mp(coPp) argFGH_Pp] (SEQ ID NO:49, see table 17).

The *P. putida* strain KT2440 was transformed with the plasmid pACYCATh-5{PRha}[agat_Mp(coPp) argFGH_Pp] by means of electroporation and plated onto LB-agar plates supplemented with tetracycline (10 mg/l). Transformants were checked for the presence of the correct plasmid by plasmid preparation and analytic restriction analysis. The resulting strain was named *P. putida* KT2440/pACYCATh-5{PRha}[agat_Mp(coPp) argFGH_Pp] (see table 18).

Table 16: Primers used for cloning of AGAT_Mp and *argFGH* into pACYCATh-5.

| Primer | SEQ ID | Template | PCR Product |
|---|---|---|---|
| MW_20_01_fw | SEQ ID NO:36 | AGAT_Mp gene fragment | AGAT_Mp |
| MW_20_03_rv | SEQ ID NO:46 | AGAT_Mp gene fragment | AGAT_Mp |
| MW_20_04_fw | SEQ ID NO:47 | argFGH gene fragment | argFGH |
| MW_20_09_rv | SEQ ID NO:48 | argFGH gene fragment | argFGH |

**Example 17: Cloning of the genes *carAB* from *P. putida* KT2440 into the expression vector pACYCATh-5{PRha} [agat_Mp(coPp) argFGH_Pp]**

For the heterologous expression of AGAT_Mp from *Moorea producens, argF* (SEQ ID NO:39), *argG* (SEQ ID NO:41), *argH* (SEQ ID NO:43), *carA* (SEQ ID NO:50) and *carB* (SEQ ID NO:52) from *P. putida* KT2440 the plasmid pACYCATh-5{PRha}[agat_Mp(coPp) argFGH_Pp]{carAB_Pp}[carAB_Pp][ter] was constructed. The *carA* (SEQ ID NO:50) and *carB* gene (SEQ ID NO:52) encoding a carbamoyl-phosphate synthase (CarAB, EC 6.3.5.5, SEQ ID NO:51 and SEQ ID NO:53) were amplified from genomic DNA of *P. putida* KT2440 including the native promoter of the *carAB* operon via PCR using the primer MW_20_35_fw (SEQ ID NO:54) and MW_20_36_rv (SEQ ID NO:55). For amplification the Phusion™ High-Fidelity Master Mix from New England Biolabs (Ipswich, USA) was used according to manufacturer's manual. The PCR product (SEQ ID NO:56) was cloned into the pACYCATh-5{PRha}[agat_Mp(coPp) argFGH_Pp] cut with *Bsu*36I using NEBuilder® HiFi DNA Assembly Cloning Kit from (New England BioLabs Inc., Ipswich, USA, Cat. No. E5520). The assembled product was transformed into 10-beta electrocompetent *E. coli* cells (New England BioLabs Inc., Ipswich, USA, Cat. No. C3020K). Procedure of PCR purification, cloning and transformation were carried out according to manufacturer's manual. The correct insertion of the target genes was checked by restriction analysis and the authenticity of the introduced DNA fragments was verified by DNA sequencing. The resulting expression vector was named pACYCATh-5{PRha}[agat_Mp(coPp) argFGH_Pp]{carAB_Pp}[carAB_Pp][ter] (SEQ ID NO:57, see table 17).

The *P. putida* strain KT2440 was transformed with the plasmid pACYCATh-5{PRha}[agat_Mp(coPp) argFGH_Pp]{carAB_Pp}[carAB_Pp][ter] by means of electroporation and plated onto LB-agar plates supplemented with tetracycline (10 mg/l). Transformants were checked for the presence of the correct plasmid by plasmid preparation and analytic restriction analysis. The resulting strain was named *P. putida* KT2440/pACYCATh-5{PRha}[agat_Mp(coPp) argFGH_Pp]{carAB_Pp}[carAB_Pp][ter] (see table 18).

Table 17: List of *P. putida* expression plasmids

| Plasmid | Comment |
|---|---|
| pACYCATh-5 | Empty *E. coli* - *P. putida* shuttle plasmid |
| pACYCATh-5{PRha}[agat_Mp(coPp)] | Expression of AGAT_Mp (*Moorea producens*) |
| pACYCATh-5{PRha}[agat_Mp(coPp) argFGH_Pp] | Expression of AGAT_Mp (*Moorea producens*), *argF, argG, argH* (*P. putida*) |
| pACYCATh-5{PRha}[agat_Mp(coPp) argFGH_Pp] {carAB_Pp}[carAB_Pp][ter] | Expression of AGAT_Mp (*Moorea producens*), *argF, argG, argH, carA, carB* (*P. putida*) |

Table 18: List of plasmid-containing *P. putida* strains

| Plasmid | Cloned gene | Recipient strain | Resulting strain |
|---|---|---|---|
| pACYCATh-5 | none | *P. putida* KT2440 | *P. putida* KT2440/pACYCATh-5 |
| pACYCATh-5{PRha}[agat_Mp(coPp)] | AGAT_Mp | *P. putida* KT2440 | *P. putida* KT2440/pACYCATh-5{PRha}[agat_Mp(coPp)] |
| pACYCATh-5{PRha}[agat_Mp(coPp) argFGH_Pp] | AGAT_Mp, *argF, argG, argH* | *P. putida* KT2440 | *P. putida* KT2440/pACYCATh-5{PRha}[agat_Mp(coPp) argFGH_Pp] |
| pACYCATh-5{PRha}[agat_Mp(coPp) argFGH_Pp]{carAB_Pp}[carAB _Pp][ter] | AGAT_Mp, *argF, argG, argH, carA, carB* | *P. putida* KT2440 | *P. putida* KT2440/pACYCATh-5{PRha}[agat_Mp(coPp) argFGH_Pp]{carAB_Pp}[carAB _Pp][ter] |

## Example 18: Impact of AGAT on GAA production in P. *putida* KT2440

Strain *P. putida* KT2440/pACYCATh-5{PRha}[agat_Mp(coPp)] carrying a gene for the AGAT enzyme from M. *producens* and *P. putida* KT2440/pACYCATh-5 (empty vector control) were analyzed for their ability to produce GAA in batch cultivation using shake flasks. On a LB agar plate containing 10 mg/l tetracycline an inoculation loop of glycerol cryoculture of the corresponding strains was streaked. The agar plates were incubated for 24 h at 30°C. A 100 ml flask with baffles containing 15 ml of seed medium (autoclaved: 4.4 g/L $Na_2HPO_4$ x 2 $H_2O$, 1.5 g/L $KH_2PO_4$, 1 g/L $NH_4Cl$, 10 g/L yeast extract, sterilized separately: 20 g/L glucose, 0.2 g/L $MgSO_4$ x 7 $H_2O$, 0.006 g/L $FeCl_3$, 0.015 g/L $CaCl_2$, 1 ml/L trace elements solution SL6 (sterile-filtered: 0.3 g/L $H_3BO_3$, 0.2 g/L $CoCl_2 \times 6\ H_2O$, 0.1 g/L $ZnSO_4 \times 7\ H_2O$, 0.03 g/L $MnCl_2 \times 4H_2O$, 0.01 g/L $CuCl_2 \times 2\ H_2O$, 0.03 g/L $Na_2MoO_4$ x 2 $H_2O$, 0.02 g/L $NiCl_2 \times 6\ H_2O$), pH 7) with 10 mg/l tetracycline was inoculated with a single colony of an agar plate and incubated in a shaking incubator for 18 h at 30°C and 200 rpm to produce a preculture. The preculture was used to inoculate 40 ml of M12 medium (composition: (2.2 g/L $(NH_4)_2SO_4$, 0.02 g/L NaCl, 0.4 g/L $MgSO_4 \times 7H_2O$, 0.04 g/L $CaCl_2 \times 2H_2O$, sterilized separately: 2 g/L $KH_2PO_4$, 8.51 g/L $Na_2HPO_4$, 20 g/L glucose, 10 ml/l trace elements solution M12 (sterile-filtered: 0.2 g/L $ZnSO_4 \times 7\ H_2O$, 0.1 g/L $MnCl_2 \times 4H_2O$, 1.5 g/L $Na_3$-citrate $\times 2\ H_2O$, 0.1 g/L $CuSO_4 \times 5\ H_2O$, 0.002 g/L $NiCl_2$ x 6 $H_2O$, 0.003 g/L $Na_2MoO_4 \times 2\ H_2O$, 0.03 g/L $H_3BO_3$, 1 g/L $FeSO_4 \times 7\ H_2O$), pH 7.4) with 10 mg/l tetracycline, 3.48 g/l arginine and 1.5 g/l glycine to a start $OD_{600}$ of 0.1. The strains were cultivated for 48 h. At an $OD_{600}$ of ~0.5-0.8 gene expression was induced by adding 0.2 % (w/v) rhamnose. 9 h and 24 h after induction 1.74 g/l arginine and 0.75 g/l glycine were spiked. At the end of cultivation samples were taken to determine the concentration of GAA produced.
The results are shown in table 19.

Table 19: GAA production by strains *P. putida* KT2440/pACYCATh-5 and *P. putida* KT2440/pACYCATh-5{PRha}[agat_Mp(coPp)]

| Strain | GAA |
|---|---|
| *P. putida* KT2440/pACYCATh-5 | not detectable |
| *P. putida* KT2440/pACYCATh-5{PRha}[agat_Mp(coPp)] | 81.5 mg/l |

As can be seen in table 19 the strain *P. putida* KT2440/pACYCATh-5{PRha}[agat_Mp(coPp)] equipped with the AGAT_Mp gene from *M. producens* was able to produce about 81.5 mg/l GAA. The control strain *P. putida* KT2440/pACYCATh-5 could not produce any GAA at all.

## Example 19: Impact of AGAT and increased L-arginine supply on GAA production in P. *putida* KT2440

Strain *P. putida* KT2440/pACYCATh-5{PRha}[agat_Mp(coPp)] carrying a gene for the AGAT enzyme from *M. producens* and strain *P. putida* KT2440/pACYCATh-5{PRha}[agat_Mp(coPp) argFGH_Pp] carrying additionally the arginine biosynthesis genes *argFGH* were analyzed for their ability to produce GAA in batch cultivation using shake flasks. On an LB agar plate containing 10 mg/l tetracycline an inoculation loop of glycerol cryoculture of the corresponding strains was streaked. The agar plate was incubated for 24 h at 30°C. A 100 ml flask with baffles containing 15 ml of seed medium (autoclaved: 4.4 g/L $Na_2HPO_4$ x 2 $H_2O$, 1.5 g/L $KH_2PO_4$, 1 g/L $NH_4Cl$, 10 g/L yeast extract, sterilized separately: 20 g/L glucose, 0.2 g/L $MgSO_4$ x 7 $H_2O$, 0.006 g/L $FeCl_3$, 0.015 g/L $CaCl_2$, 1 ml/L trace elements solution SL6 (sterile-filtered: 0.3 g/L $H_3BO_3$, 0.2 g/L $CoCl_2 \times 6 H_2O$, 0.1 g/L $ZnSO_4$ x 7 $H_2O$, 0.03 g/L $MnCl_2$ x $4H_2O$, 0.01 g/L $CuCl_2 \times 2 H_2O$, 0.03 g/L $Na_2MoG_4$ x 2 $H_2O$, 0.02 g/L $NiCl_2 \times 6 H_2O$), pH 7) with 10 mg/l tetracycline was inoculated with a single colony of an agar plate and incubated in a shaking incubator for 18 h at 30°C and 200 rpm to produce a preculture. The preculture was used to inoculate 40 ml of M12 medium (composition: (2.2 g/L $(NH_4)_2SO_4$, 0.02 g/L NaCl, 0.4 g/L $MgSO_4 \times 7H_2O$, 0.04 g/L $CaCl \times 2H_2O$, sterilized separately: 2 g/L $KH_2PO_4$, 8.51 g/L $Na_2HPO_4$, 20 g/L glucose, 10 ml/l trace elements solution M12 (sterile-filtered: 0.2 g/L $ZnSO_4$ x 7 $H_2O$, 0.1 g/L $MnCl_2 \times 4H_2O$, 1.5 g/L $Na_3$-citrate $\times 2 H_2O$, 0.1 g/L $CuSO_4 \times 5 H_2O$, 0.002 g/L $NiCl_2 \times 6 H_2O$, 0.003 g/L $Na_2MoG_4$ x 2 $H_2O$, 0.03 g/L $H_3BO_3$, 1 g/L $FeSO_4$ x 7 $H_2O$), pH 7.4) with 10 mg/l tetracycline, 3.48 g/l arginine and 1.5 g/l glycine to a start $OD_{600}$ of 0.1. The strains were cultivated for 48 h. At an $OD_{600}$ of ~0.5-0.8 gene expression was induced by adding 0.2 % (w/v) rhamnose. 9 h and 24 h after induction 1.74 g/l arginine and 0.75 g/l glycine were spiked. At the end of cultivation samples were taken to determine the concentration of GAA produced.

The results are shown in table 20.

Table 20: GAA production by strains *P. putida* KT2440/pACYCATh-5{PRha}[agat_Mp(coPp)] and *P. putida* KT2440/pACYCATh-5{PRha}[agat_Mp(coPp) argFGH_Pp]

| Strain | GAA |
|---|---|
| *P. putida* KT2440/pACYCATh-5 | not detectable |
| *P. putida* KT2440/pACYCATh-5{PRha}[agat_Mp(coPp)] | 81.5 mg/l |
| *P. putida* KT2440/pACYCATh-5{PRha}[agat_Mp(coPp) argFGH_Pp] | 169.5 mg/l |

As can be seen in table 20 the strain *P. putida* KT2440/pACYCATh-5{PRha}[agat_Mp(coPp)] equipped with the AGAT_Mp gene from M. *producens* was able to produce about 81.5 mg/l GAA. Additionally implementation of *argF, argG* and *argH* in strain *P. putida* KT2440/pACYCATh-5{PRha}[agat_Mp(coPp) argFGH_Pp] improved GAA production to 169.5 mg/l.

**Example 20: Impact of AGAT and of L-arginine regeneration on GAA production in *P. putida* KT2440**

Strain *P. putida* KT2440/pACYCATh-5{PRha}[agat_Mp(coPp)] carrying a gene for the AGAT enzyme from *M. producens,* strain *P. putida* KT2440/pACYCATh-5{PRha}[agat_Mp argFGH_Pp] carrying additionally the arginine biosynthesis genes *argFGH* and strain *P. putida* KT2440/pACYCATh-5{PRha}[agat_Mp(coPp) argFGH_Pp]{carAB_Pp} [carAB_Pp][ter] carrying additionally the carbamoyl-phosphate synthase genes *carAB* were analyzed for their ability to produce GAA in batch cultivation using shake flasks. The conversion of L-ornithine to L-citrulline, catalyzed by the ornithine carbamoyltransferase, depends on the availability of the co-substrate carbamoyl phosphate. Carbamoyl phosphate is produced by the carbamoyl phosphate synthase, which is encoded by the genes *carA* and *carB*. On an LB agar plate containing 10 mg/l tetracycline an inoculation loop of glycerol cryoculture of the corresponding strains was streaked. The agar plate was incubated for 24 h at 30°C. A 100 ml flask with baffles containing 15 ml of seed medium (autoclaved: 4.4 g/L $Na_2HPO_4$ x 2 $H_2O$, 1.5 g/L $KH_2PO_4$, 1 g/L $NH_4Cl$, 10 g/L yeast extract, sterilized separately: 20 g/L glucose, 0.2 g/L $MgSO_4$ x 7 $H_2O$, 0.006 g/L $FeCl_3$, 0.015 g/L $CaCl_2$, 1 ml/L trace elements solution SL6 (sterile-filtered: 0.3 g/L $H_3BO_3$, 0.2 g/L $CoCl_2 \times 6 H_2O$, 0.1 g/L $ZnSO_4$ x 7 $H_2O$, 0.03 g/L $MnCl_2$ x $4H_2O$, 0.01 g/L $CuCl_2 \times 2 H_2O$, 0.03 g/L $Na_2MoG_4$ $\times 2 H_2O$, 0.02 g/L $NiCl_2 \times 6 H_2O$), pH 7) with 10 mg/l tetracycline was inoculated with a single colony of an agar plate and incubated in a shaking incubator for 18 h at 30°C and 200 rpm to produce a preculture. The preculture was used to inoculate 40 ml of M12 medium (composition: (2.2 g/L $(NH_4)_2SO_4$, 0.02 g/L NaCl, 0.4 g/L $MgSO_4 \times 7H_2O$, 0.04 g/L $CaCl_2 \times 2H_2O$, sterilized separately: 2 g/L $KH_2PO_4$, 8.51 g/L $Na_2HPO_4$, 20 g/L glucose, 10 ml/l trace elements solution M12 (sterile-filtered: 0.2 g/L $ZnSO_4$ x 7 $H_2O$, 0.1 g/L $MnCl_2 \times 4H_2O$, 1.5 g/L $Na_3$-citrate $\times 2 H_2O$, 0.1 g/L $CuSO_4$ x 5 $H_2O$, 0.002 g/L $NiCl_2 \times 6 H_2O$, 0.003 g/L $Na_2MoG_4$ x 2 $H_2O$, 0.03 g/L $H_3BO_3$, 1 g/L $FeSO_4$ x 7 $H_2O$), pH 7.4) with 10 mg/l tetracycline, 3.48 g/l arginine and 1.5 g/l glycine to a start $OD_{600}$ of 0.1. The strains were cultivated for 48 h. At an $OD_{600}$ of ~0.5-0.8 gene expression was induced by adding 0.2 % (w/v) rhamnose. 4 h / 18 h / 23 h after induction 6.97 g/l Arg/1.5 g/l Gly, 2.34 g/L Arg/0.75 g/L Gly and 6.97 g/l Arg/1.5 g/l Gly were spiked. At the end of cultivation samples were taken to determine the concentration of GAA produced.

The results are shown in table 21.

Table 21: GAA production by strains *P. putida* KT2440/pACYCATh-5{PRha}[agat_Mp(coPp)], *P. putida* KT2440/pACYCATh-5{PRha}[agat_Mp(coPp) argFGH_Pp] and *P. putida* KT2440/pACYCATh-5{PRha}[agat_Mp (coPp) argFGH_Pp]{carAB_Pp}[carAB_Pp][ter]

| Strain | GAA |
| --- | --- |
| *P. putida* KT2440/pACYCATh-5 | not detectable |
| *P. putida* KT2440/pACYCATh-5{PRha}[agat_Mp argFGH_Pp] | 589 mg/l |
| *P. putida* KT2440/pACYCATh-5{PRha}[agat_Mp(coPp) argFGH_Pp]{carAB_Pp}[carAB_Pp][ter] | 693 mg/l |

As can be seen in table 21 the strain equipped with the AGAT_Mp gene from *M. producens* and the *argFGH* genes from *P. putida* was able to produce 589 mg/l GAA. Additionally implementation of *carAB* in strain *P. putida* KT2440/pACYCATh-5{PRha}[agat_Mp(coPp) argFGH_Pp]{carAB_Pp}[carAB_Pp][ter] improved GAA production to 693 mg/l.

**Claims**

1. A microorganism comprising an increased activity of an enzyme having the function of a carbamoylphosphate synthase compared to the respective enzymic activity in the wildtype microorganism and comprising at least one heterologous gene coding for a protein having the activity of a L-arginine:glycine amidinotransferase, wherein the increased activity of the enzyme having the function of a carbamoylphosphate synthase is achieved by overexpression of a gene coding for the enzyme having the function of a carbamoylphosphate synthase.

2. The microorganism of claim 1, wherein the overexpression of the gene coding for the enzyme having the function of a carbamoylphosphate synthase is achieved by increasing the copy number of the gene and/or by functionally linking the gene with a strong promoter.

3. The microorganism of claim 1 or claim 2, wherein the activity of a L-arginine:glycine amidinotransferase is increased by overexpression of the gene coding for the L-arginine:glycine amidinotransferase.

4. The microorganism of any of the preceding claims having an improved ability to produce L-arginine compared with the ability of the wildtype microorganism.

5. The microorganism of claim 4, wherein the *argR* gene coding for the arginine responsive repressor protein ArgR is attenuated or deleted.

6. The microorganism of any of claims 4 or 5, wherein at least one or more of the genes coding for an enzyme of the biosynthetic pathway of L-ornithine and L-arginine, comprising *gdh, argJ, argB, argC* and/or *argD* coding for a glutamate dehydrogenase, an ornithine acetyltransferase, an acetylglutamate kinase, an acetylglutamylphosphate reductase and for an acetylornithine aminotransferase, respectively, is overexpressed.

7. The microorganism of any of claims 1 to 6, wherein the protein having the function of an L-arginine:glycine amidinotransferase comprises an amino acid sequence which is at least 70 % identical to the amino acid sequence selected from the group consisting of SEQ ID NO: 2 SEQ ID NO: 16, SEQ ID NO: 24 and SEQ ID NO: 20.

8. The microorganism of any of the preceding claims, wherein the microorganism belongs to the genus *Corynebacterium,* to the genus *Enterobacteriaceae* or to the genus *Pseudomonas.*

9. The microorganism of claim 9, wherein the microorganism is *Corynebacterium glutamicum.*

10. The microorganism of claim 8 wherein the microorganism is *Escherichia coli.*

11. The microorganism of claim 8 wherein the microorganism is *Pseudomonas putida.*

**12.** A method for the fermentative production of guanidino acetic acid (GAA), comprising the steps of a) cultivating the microorganism as defined in any of the preceding claims in a fermentation medium, and b) accumulating GAA in the medium to form a GAA containing fermentation broth.

**13.** The method of claim 12 further comprising isolating GAA from the GAA containing fermentation broth.

**14.** A microorganism as claimed in any of claims 1 to 11, further comprising a gene coding for an enzyme having the activity of a guanidinoacetate N-methyltransferase.

**15.** The microorganism of claim 14, wherein the gene coding for an enzyme having the activity of a guanidinoacetate N-methyltransferase is overexpressed.

**16.** A method for the fermentative production of creatine, comprising the steps of a) cultivating the microorganism as defined in claim 14 or claim 15 in a fermentation medium, and b) accumulating creatine in the medium to form a creatine containing fermentation broth.

**17.** The method of claim 16, further comprising isolating creatine from the creatine containing fermentation broth.


**Patentansprüche**

**1.** Mikroorganismus, umfassend eine erhöhte Aktivität eines Enzyms mit der Funktion einer Carbamoylphosphat-Synthase im Vergleich zur entsprechenden Enzymaktivität im Wildtyp-Mikroorganismus und umfassend wenigstens ein heterologes Gen, das für ein Protein mit der Aktivität einer L-Arginin:Glycin-Amidinotransferase codiert, wobei die erhöhte Aktivität des Enzyms mit der Funktion einer Carbamoylphosphat-Synthase durch Überexpression eines für das Enzym mit der Funktion einer Carbamoylphosphat-Synthase codierenden Gens erzielt wird.

**2.** Mikroorganismus nach Anspruch 1, wobei die Überexpression des für das Enzym mit der Funktion einer Carbamoylphosphat-Synthase codierenden Gens durch Erhöhen der Kopienzahl des Gens und/oder durch funktionelles Verknüpfen des Gens mit einem starken Promotor erzielt wird.

**3.** Mikroorganismus nach Anspruch 1 oder Anspruch 2, wobei die Aktivität einer L-Arginin:Glycin-Amidinotransferasedurch Überexpression des für die L-Arginin:Glycin-Amidinotransferasecodierenden Gens erhöht wird.

**4.** Mikroorganismus nach einem der vorhergehenden Ansprüche, aufweisend eine verbesserte Fähigkeit, L-Arginin zu produzieren, gegenüber der Fähigkeit des Wildtyp-Mikroorganismus.

**5.** Mikroorganismus nach Anspruch 4, wobei das für das auf Arginin ansprechende Repressorprotein ArgR codierende *argR*-Gen abgeschwächt oder deletiert ist.

**6.** Mikroorganismus nach einem der Ansprüche 4 oder 5, wobei wenigstens eines oder mehrere der für ein Enzym des Biosynthesewegs von L-Ornithin und L-Arginin codierenden Gene, die *gdh, argJ, argB, argC* und/oder *argD* umfassen, die für eine Glutamat-Dehydrogenase, eine Ornithin-Acetyltransferase, eine Acetylglutamat-Kinase, eine Acetylglutamylphosphat-Reduktase bzw. für eine Acetylornithin-Aminotransferase codieren, überexprimiert wird bzw. werden.

**7.** Mikroorganismus nach einem der Ansprüche 1 bis 6, wobei das Protein mit der Funktion einer L-Arginin:Glycin-Amidinotransferaseeine Aminosäuresequenz umfasst, die zu wenigstens 70 % mit der aus der Gruppe bestehend aus SEQ ID NO: 2 SEQ ID NO: 16, SEQ ID NO: 24 und SEQ ID NO: 20 ausgewählten Aminosäuresequenz identisch ist.

**8.** Mikroorganismus nach einem der vorhergehenden Ansprüche, wobei der Mikroorganismus zur Gattung *Corynebacterium,* zur Gattung *Enterobacteriaceae* oder zur Gattung *Pseudomonas* gehört.

**9.** Mikroorganismus nach Anspruch 9, wobei es sich bei dem Mikroorganismus um *Corynebacterium glutamicum* handelt.

**10.** Mikroorganismus nach Anspruch 8, wobei es sich bei dem Mikroorganismus um *Escherichia coli* handelt.

**11.** Mikroorganismus nach Anspruch 8, wobei es sich bei dem Mikroorganismus um *Pseudomonas putida* handelt.

**12.** Verfahren zur fermentativen Herstellung von Guanidinoessigsäure (Guanidino Acetic Acid, GAA), umfassend die Schritte a) Kultivieren des Mikroorganismus gemäß einem der vorhergehenden Ansprüche in einem Fermentationsmedium und b) Anreichern von GAA im Medium unter Bildung einer GAA-haltigen Fermentationsbrühe.

**13.** Verfahren nach Anspruch 12, ferner umfassend Isolieren von GAA aus der GAA-haltigen Fermentationsbrühe.

**14.** Mikroorganismus gemäß einem der Ansprüche 1 bis 11, ferner umfassend ein Gen, das für ein Enzym mit der Aktivität einer Guanidinoacetat-N-Methyltransferase codiert.

**15.** Mikroorganismus nach Anspruch 14, wobei das für ein Enzym mit der Aktivität einer Guanidinoacetat-N-Methyltransferase codierende Gen überexprimiert wird.

**16.** Verfahren zur fermentativen Herstellung von Creatin, umfassend die Schritte a) Kultivieren des Mikroorganismus gemäß Anspruch 14 oder Anspruch 15 in einem Fermentationsmedium und b) Anreichern von Creatin im Medium unter Bildung einer creatinhaltigen Fermentationsbrühe.

**17.** Verfahren nach Anspruch 16, ferner umfassend Isolieren von Creatin aus der creatinhaltigen Fermentationsbrühe.

**Revendications**

**1.** Micro-organisme comprenant une activité accrue d'une enzyme ayant la fonction d'une carbamoylphosphate synthase par rapport à l'activité enzymatique respective dans le micro-organisme de type sauvage et comprenant au moins un gène hétérologue codant pour une protéine ayant l'activité d'une L-arginine:glycine amidinotransférase, dans lequel l'activité accrue de l'enzyme ayant la fonction d'une carbamoylphosphate synthase est obtenue par surexpression d'un gène codant pour l'enzyme ayant la fonction d'une carbamoylphosphate synthase.

**2.** Micro-organisme selon la revendication 1, dans lequel la surexpression du gène codant pour l'enzyme ayant la fonction d'une carbamoylphosphate synthase est obtenue en augmentant le nombre de copies du gène et/ou en liant de manière fonctionnelle le gène à un promoteur fort.

**3.** Micro-organisme selon la revendication 1 ou la revendication 2, dans lequel l'activité d'une L-arginine: glycine amidinotransférase est augmentée par surexpression du gène codant pour la L-arginine:glycine amidinotransférase.

**4.** Micro-organisme selon l'une quelconque des revendications précédentes ayant une capacité améliorée à produire de la L-arginine par rapport à la capacité du micro-organisme de type sauvage.

**5.** Micro-organisme selon la revendication 4, dans lequel le gène *argR* codant pour la protéine de répression réactive à l'arginine ArgR est atténué ou délété.

**6.** Micro-organisme selon l'une quelconque des revendications 4 ou 5, dans lequel au moins l'un ou plusieurs des gènes codant pour une enzyme de la voie biosynthétique de la L-ornithine et de la L-arginine, comprenant *gdh, argJ, argB, argC* et/ou *argD* codant pour une glutamate déshydrogénase, une ornithine acétyltransférase, une acétylglutamate kinase, une acétylglutamylphosphate réductase et pour une acétylornithine aminotransférase, respectivement, est surexprimé.

**7.** Micro-organisme selon l'une quelconque des revendications 1 à 6, dans lequel la protéine ayant la fonction d'une L-arginine:glycine amidinotransférase comprend une séquence d'acides aminés qui est au moins 70 % identique à la séquence d'acides aminés sélectionnée dans le groupe constitué par les SEQ ID NO: 2, SEQ ID NO: 16, SEQ ID NO: 24 et SEQ ID NO: 20.

**8.** Micro-organisme selon l'une quelconque des revendications précédentes, dans lequel le micro-organisme appartient au genre *Corynebacterium,* au genre *Enterobacteriaceae* ou au genre *Pseudomonas.*

**9.** Micro-organisme selon la revendication 9, dans lequel le micro-organisme est *Corynebacterium glutamicum.*

**10.** Micro-organisme selon la revendication 8 dans lequel le micro-organisme est *Escherichia coli.*

**11.** Micro-organisme selon la revendication 8 dans lequel le micro-organisme est *Pseudomonas putida.*

**12.** Procédé pour la production par fermentation d'acide guanidino acétique (GAA), comprenant les étapes de a) culture du micro-organisme tel que défini dans l'une des revendications précédentes dans un milieu de fermentation, et b) accumulation de GAA dans le milieu pour former un bouillon de fermentation contenant du GAA.

**13.** Procédé selon la revendication 12 comprenant en outre l'isolement de GAA du bouillon de fermentation contenant du GAA.

**14.** Micro-organisme selon l'une quelconque des revendications 1 à 11, comprenant en outre un gène codant pour une enzyme ayant l'activité d'une guanidinoacétate N-méthyltransférase.

**15.** Micro-organisme selon la revendication 14, dans lequel le gène codant pour une enzyme ayant l'activité d'une guanidinoacétate N-méthyltransférase est surexprimé.

**16.** Procédé de production par fermentation de créatine, comprenant les étapes de a) culture du micro-organisme tel que défini dans la revendication 14 ou la revendication 15 dans un milieu de fermentation, et b) accumulation de créatine dans le milieu pour former un bouillon de fermentation contenant de la créatine.

**17.** Procédé selon la revendication 16, comprenant en outre l'isolement de créatine du bouillon de fermentation contenant de la créatine.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2005120246 A1
- US 2011257075 A1
- US 20060200870 A1
- WO 2018079687 A1, Mijts
- US 3849250 A, Nakayama and Yoshida
- EP 1057893 A1, Kurahashi
- US 7160705 B2, Suga
- EP 3153573 A1, Bae
- WO 2006057450 A1
- CN 106065411 A

### Non-patent literature cited in the description

- **HUMM et al.** *Biochem. J.,* 1997, vol. 322, 771-776
- **GUTHMILLER et al.** *J Biol Chem.,* 01 July 1994, vol. 269 (26), 17556-60
- **MUENCHHOFF et al.** *FEBS Journal,* 2010, vol. 277, 3844-3860
- **SOSIO et al.** *Cell Chemical Biology,* 17 May 2018, vol. 25, 540-549
- **HUMM.** Escherichia coli. *Biochem. J.,* 1997, vol. 322, 771-776
- **MARC et al.** *Eur. J. Biochem.,* vol. 267, 5217-5226, 200
- **PARK et al.** *NATURE COMMUNICATIONS*
- **YIM et al.** *J Ind Microbiol Biotechnol,* 2011, vol. 38, 1911-1920
- **GINESY et al.** *Microbial Cell Factories,* 2015, vol. 14, 29
- **SAKANYAN et al.** *Microbiology,* 1996, vol. 142, 9-108
- **MARCHLER-BAUERA et al.** CDD/SPARCLE: functional classification of proteins via subfamily domain architectures. *Nucleic Acids Res.,* 2017, vol. 45 (D1), D200-D203
- **PISSOWOTZKI K et al.** *Mol Gen Genet,* 1991, vol. 231, 113-123
- **D'HOOGHE I et al.** *J Bacteriol,* 1997, vol. 179, 7403-7409
- **KANAOKA M et al.** *Jpn J Cancer Res,* 1987, vol. 78, 1409-1414
- Glycine amidinotransferase. *Database UniProt,* 15 February 2017
- **M. PATEK et al.** *Microbial Biotechnology,* 2013, vol. 6, 103-117
- **SANGER et al.** *Proceedings of the National Academy of Sciences USA,* 1977, vol. 74, 5463-5467
- **LEAO T ; CASTELÃO G ; KOROBEYNIKOV A ; MONROE EA ; PODELL S ; GLUKHOV E ; ALLEN EE ; GERWICK WH ; GERWICK L.** *Proc Natl Acad Sci USA.,* 21 March 2017, vol. 114 (12), 3198-3203
- **MIHALI TK ; KELLMANN R ; MUENCHHOFF J ; BARROW KD ; NEILAN BA.** Characterization of the gene cluster responsible for cylindrospermopsin biosynthesis. *Appl Environ Microbiol.,* 2008, vol. 74 (3), 716-22
- **HUMM A ; HUBER R ; MANN K.** The amino acid sequences of human and pig l-arginine:glycine amidinotransferase. *FEBS Letters,* 1994, vol. 339 (1-2), 101-107
- **HUMM A ; FRITSCHE E ; MANN K ; GÖHL M ; HUBER R.** Recombinant expression and isolation of human L-arginine : glycine amidinotransferase and identification of its active-site cysteine residue. *Biochem. J.,* 1997, vol. 322, 771-776
- **HANSTED JG ; PIETIKÄINEN L ; HÖG F ; SPERLING-PETERSEN HU ; MORTENSEN KK.** Expressivity tag: A novel tool for increased expression in Escherichia coli. *Journal of Biotechnology,* 2011, vol. 155 (2011), 275-283
- **EIKMANNS BJ ; KLEINERTZ E ; LIEBL W ; SAHM H.** A family of Corynebacterium glutamicum/Escherichia coli shuttle vectors for cloning, controlled gene expression, and promoter probing. *Gene,* 15 June 1991, vol. 102 (1), 93-8
- **BLATTNER FR ; PLUNKETT G 3RD ; BLOCH CA ; PERNA NT ; BURLAND V ; RILEY M ; COLLADO-VIDES J ; GLASNER JD ; RODE CK ; MAYHEW GF.** *Science,* 05 September 1997, vol. 277 (5331), 1453-62
- **KINOSHITA S ; UDAKA S ; SHIMONO M.** *J. Gen. Appl. Microbiol.,* 1957, vol. 3 (3), 193-205
- **PARK SH ; KIM HU ; KIM TY ; PARK JS ; KIM SS ; LEE.** *Nat Commun.,* 05 August 2014
- **KINOSHITA et al.** *J. Gen. Appl. Microbiol.,* 1957, vol. 3 (3), 193-205

- **PARK et al.** *Nat Commun.,* 05 August 2014, vol. 5, 4618

- **ITOH Y ; WATSON JM ; HAAS D ; LEISINGER T.** *Plasmid,* 1984, vol. 11 (3), 206-20